Europäisches Patentamt
European Patent Office
Office européen des brevets

(19)

(11) Veröffentlichungsnummer: **0 233 837 A2**

(12)

# EUROPÄISCHE PATENTANMELDUNG

(21) Anmeldenummer: **87810034.6**

(22) Anmeldetag: **19.01.87**

(51) Int. Cl.4: **C 07 H 15/04**
C 07 H 15/14, A 61 K 31/70

(30) Priorität: **24.01.86 CH 276/86**

(43) Veröffentlichungstag der Anmeldung:
**26.08.87 Patentblatt 87/35**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Fechtig, Bruno Dr.**
**Hinterlindenweg 1**
**CH-4153 Reinach (CH)**

**Baschang, Gerhard Dr.**
**Bückenweg 7**
**CH-4126 Bettingen (CH)**

## (54) Saccharidderivate und Verfahren zu ihrer Herstellung.

(57) Beschrieben sind Mono- und Disaccharidylderivate, die über ein Brückenglied mit einem Kephalinderivat verknüpft sind, der Formel I und Verfahren zu ihrer Herstellung.

$$R^1-X-Y-\overset{O}{\overset{\|}{C}}-NH-\overset{R^2}{\overset{|}{C}H}-CH_2-O-\underset{HO}{\overset{O}{\overset{\|}{P}}}-O-\underset{R^4}{\overset{R^3}{\overset{|}{C}H}} \qquad (I)$$

Darin bedeutet $R_1$ a) Aldohexosyl, b) D-Aldohexosyl, welches in 4-oder 6-Stellung glykosidisch mit D-Aldohexosyl verknüpft ist, c) Aldopentosyl, d) 6-Desoxy-aldohexosyl oder e) 2-Acetylamino-2-desoxy-D-aldohexosyl, wobei in den oben unter a) bis e) genannten Resten vorhandene freie Hydroxygruppen peracetyliert sein können. X bedeutet Sauerstoff oder Schwefel. Y bedeutet Alkylen mit bis zu 10 C-Atomen, worin 1-3 nicht-endständige Methylengruppen durch Sauerstoff, Carbonylimino oder Carbonyloxy ersetzt sein können. $R^2$ bedeutet Wasserstoff, Carboxy, Niederalkoxycarbonyl, Benzyloxycarbonyl oder Carbamoyl. $R^3$ bedeutet Wasserstoff und $R^4$ bedeutet eine 1,2-Dihydroxy-ethyl-, 2-Hydroxy-ethyl- oder Hydroxymethyl-Gruppe, in der mindestens eine Hydroxygruppe mit einer unsubstituierten aliphatischen $C_{10-24}$-Carbonsäure verestert ist, und in der die andere Hydroxygruppe, falls vorhanden, frei oder mit einer aliphatischen $C_{2-24}$-Carbonsäure verestert ist, oder $R^3$ und $R^4$ bedeuten je eine mit einer unsubstituierten aliphatischen $C_{10-24}$-Carbonsäure veresterte Hydroxymethylgruppe. Diese Verbindungen können als Arzneimittel, z.B. zur Prophylaxe und Therapie von Virusinfektionen, verwendet werden.

EP 0 233 837 A2

**Beschreibung**

Saccharidderivate und Verfahren zu ihrer Herstellung

Die Erfindung betrifft Mono- und Disaccharidylderivate, die über ein Brückenglied mit einem Kephalinderivat verknüpft sind, Verfahren zu ihrer Herstellung, pharmazeutische Präparate, enthaltend diese Derivate, und deren Verwendung als Arzneimittel.

Die Erfindung betrifft insbesondere Saccharidderivate der Formel I,

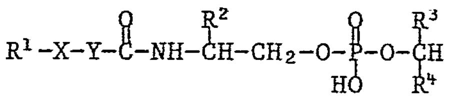

(I)

worin $R_1$ a) Aldohexosyl, b) D-Aldohexosyl, welches in 4- oder 6-Stellung glykosidisch mit D-Aldohexosyl verknüpft ist, c) Aldopentosyl, d) 6-Desoxy-aldohexosyl oder e) 2-Acetylamino-2-desoxy-D-aldohexosyl, wobei in den oben unter a) bis e) genannten Resten vorhandene freie Hydroxygruppen peracetyliert sein können, bedeutet, X Sauerstoff oder Schwefel bedeutet, Y Alkylen mit bis zu 10 C-Atomen, worin 1-3 nicht-endständige Methylengruppen durch Sauerstoff, Carbonylimino oder Carbonyloxy ersetzt sein können, bedeutet, $R^2$ Wasserstoff, Carboxy, Niederalkoxycarbonyl, Benzyloxycarbonyl oder Carbamoyl bedeutet, $R^3$ Wasserstoff und $R^4$ eine 1,2-Dihydroxy-ethyl-, 2-Hydroxy-ethyl- oder Hydroxymethyl-Gruppe, in der mindestens eine Hydroxygruppe mit einer unsubstituierten aliphatischen $C_{10-24}$-Carbonsäure verestert ist, und in der die andere Hydroxygruppe, falls vorhanden, frei oder mit einer aliphatischen $C_{2-24}$-Carbonsäure verestert ist, oder $R^3$ und $R^4$ je eine mit einer unsubstituierten aliphatischen $C_{10-24}$-Carbonsäure veresterte Hydroxymethylgruppe bedeuten, und Salze dieser Verbindungen.

Aldohexosyl $R^1$ ist L- oder vorzugsweise D-Aldohexosyl, insbesondere D-Glucosyl, D-Mannosyl oder D-Galactosyl, vorzugsweise β-D-Glucopyranosyl, α-D-Mannopyranosyl oder β-D-Galactopyranosyl. D-Aldohexosyl, welches in 4- oder 6-Stellung glykosidisch mit D-Aldohexosyl verknüpft ist, ist vorzugsweise D-Cellobiosyl, D-Lactosyl oder D-Maltosyl, insbesondere β-D-Cellobiopyranosyl, β-D-Lactopyranosyl oder β-D-Maltopyranosyl.

Aldopentosyl $R^1$ ist D-Aldopentosyl, z.B. D-Arabinosyl, oder L-Aldopentosyl, z.B. L-Arabinosyl.

6-Desoxy-aldohexosyl $R^1$ ist D-Fucosyl oder vorzugsweise L-Fucosyl, insbesondere β-L-Fucopyranosyl.

2-Acetylamino-2-desoxy-D-aldohexosyl $R^1$ ist z.B. 2-Acetylamino-2-desoxy-D-glucosyl, vorzugsweise 2-Acetylamino-2-desoxy-β-D-glucopyranosyl.

Als Beispiele für die obengenannten Reste $R^1$, worin freie Hydroxygruppen peracetyliert sind, seien insbesondere 2,3,4,6-Tetra-O-acetyl-D-glucosyl und 2,3,4,6-Tetra-O-acetyl-D-mannosyl genannt, z.B. 2,3,4,6-Tetra-O-acetyl-β-D-glucopyranosyl und 2,3,4,6-Tetra-O-acetyl-α-D-mannopyranosyl.

In den obengenannten Hexosyl- und Pentosylresten $R^1$ geht die freie Valenz vom C-Atom in 1-Stellung aus.

Alkylen Y ist verzweigt, wobei die beiden freien Valenzen von beliebigen C-Atomen ausgehen können, oder vorzugsweise unverzweigt (geradkettig). Vorzugsweise hat Alkylen Y bis zu 6, insbesondere 1-5, z.B. 3-5, C-Atome und ist z.B. Methylen oder Trimethylen. Alkylen Y, worin 1-3 nicht-endständige Methylengruppen, z.B. eine Methylengruppe, durch Sauerstoff, Carbonylimino oder Carbonyloxy ersetzt sind, ist z.B. 2,5-Dioxahexamethylen, $-CH_2-CH_2-CH_2-C(=O)-NH-CH_2-$ oder $-CH_2-CH_2-CH_2-C(=O)-O-CH_2-$. Dabei bedeutet Carbonylimino vorzugsweise die Gruppe -CO-NH-, aber auch die Gruppe -NH-CO-, und Carbonyloxy bedeutet vorzugsweise die Gruppe -CO-O-, aber auch die Gruppe -O-CO-.

Niederalkoxycarbonyl $R^2$ ist z.B. Methoxycarbonyl.

Die Konfiguration am C($-R^2$)-Atom ist (D) oder vorzugsweise (L).

Eine unsubstituierte aliphatische $C_{10-24}$-Carbonsäure ist verzweigt oder vorzugsweise unverzweigt, hat eine ungerade oder vorzugsweise eine gerade Anzahl von C-Atomen und ist insbesondere eine Alkan-oder Alkensäure mit 10-24 C-Atomen, z.B. Caprinsäure (n-Decansäure), Laurinsäure, Myristinsäure, Palmitinsäure, Stearinsäure, Arachinsäure (n-Eicosansäure) oder Oelsäure. Bevorzugt sind Alkansäuren mit 10 bis 20, insbesondere 12 bis 18, C-Atomen und Alkensäuren mit 18 C-Atomen und 1-3 isolierten Doppelbindungen, insbesondere in der Natur vorkommende Fettsäuren. Eine aliphatische $C_{2-24}$-Carbonsäure ist insbesondere eine Niederalkansäure, z.B. Essigsäure, oder eine der obengenannten $C_{10-24}$-Carbonsäuren.

Zur Kennzeichnung der Konfiguration am mittleren C-Atom des substituierten Glycerinteils von Verbindungen der Formel I, worin $R^3$ Wasserstoff und $R^4$ eine 1,2-Dihydroxy-ethyl-Gruppe bedeuten, in der mindestens eine Hydroxygruppe verestert ist, wird den IUPAC Regeln entsprechend die stereospezifische Numerierung der C-Atome des Glycerinteils, gekennzeichnet durch das Präfix "sn", verwendet. Das oberste C-Atom in der Fischer Projektion der vertikalen Kohlenstoffkette, wobei die Hydroxygruppe am C-2 nach links steht, erhält die Nummer 1. Bei den genannten erfindungsgemässen Verbindungen ist die Phosphorsäuregruppe an das C-Atom 3 im Sinne der stereospezifischen Numerierung gebunden.

Das über Sauerstoff an das Phosphoratom gebundene Proton ist sauer und kann leicht mit Basen abgespalten und durch ein anderes Kation ersetzt werden. Bei einem pH-Wert von 7 liegen die Verbindungen der Formel I daher vollständig oder zum überwiegenden Teil in Salzform vor. Diese Salze sowie die Säure-Salz-Gemische gehören ebenfalls zum Gegenstand der Erfindung. Die Verbindungen der Formel I,

worin $R^2$ Carboxy bedeutet, können Mono- oder Disalze bilden. Insbesondere betrifft die Erfindung pharmazeutisch verwendbare, nicht-toxische Salze der Verbindungen der Formel I. Es sind dies in erster Linie Metall- oder Ammoniumsalze, wie Alkalimetall- und Erdalkalimetallsalze, z.B. Natrium-, Kalium-, Magnesium- oder Calciumsalze, oder Salze mit Ammoniak oder geeigneten organischen Aminen, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung in Frage kommen, wie Niederalkylamine, z.B. Triethylamin, Hydroxyniederalkylamine, z.B. 2-Hydroxy-ethylamin, Bis-(2-hydroxyethyl)-amin, 2-Hydroxy-ethyl-diethyl-amin oder Tri-(2-hydroxyethyl)-amin, basische aliphatische Ester von Carbonsäuren, z.B. 4-Amino-benzoesäure-2-diethylaminoethylester, Niederalkylenamine, z.B. 1-Ethylpiperidin, Cycloalkylamine, z.B. Dicyclohexylamin, oder Benzylamine, z.B. N,N'-Dibenzylethylendiamin, ferner Basen vom Pyridintyp, z.B. Pyridin, Collidin oder Chinolin.

Zur Isolierung oder Reinigung können auch pharmazeutisch ungeeignete Salze Verwendung finden. Zur therapeutischen Anwendung gelangen jedoch nur die pharmazeutisch verwendbaren, nicht-toxischen Salze, die deshalb bevorzugt werden.

Die vor- und nachstehend verwendeten Allgemeinbegriffe haben vorzugsweise folgende Bedeutungen:

Das Präfix "Nieder" bezeichnet Reste bis und mit 7, insbesondere bis und mit 4, Kohlenstoffatomen und bezieht sich bei zusammengesetzten Resten nur auf den unmittelbar nachfolgenden Rest, d.h. z.B. im Falle von Niederalkoxycarbonyl nur auf Alkoxy. Unter Einschluss der Carbonylgruppe kann Niederalkoxycarbonyl also bis zu 8 C-Atome aufweisen.

Halogen ist insbesondere Chlor oder Brom, ferner Fluor oder Iod. Die Verbindungen der Formel I weisen wertvolle pharmakologische Eigenschaften auf. Erfindungsgemäss wurde überraschenderweise gefunden, dass die obengenannten Verbindungen der Formel I und ihre pharmazeutisch verwendbaren Salze sowohl zur Prophylaxe als auch zur Therapie von Virusinfektionen hervorragend geeignet sind, wie sich z.B. aus Tierversuchen, wie sie im Beispielteil exemplifiziert sind, ergibt. In diesen Tirversuchen werden Tiere, wie Mäuse oder Meerschweinchen, mit den verschiedensten Virusarten in einer Dosis, die für alle oder die grosse Mehrzahl der unbehandelten (Kontroll)Tiere letal ist, z.B. $LD_{80-90}$, infiziert und der Infektionsverlauf bei den unbehandelten Kontrolltieren im Vergleich zu Tieren beobachtet, die vor, gleichzeitig mit oder nach der Infektion mit einer der obengenannten Verbindungen oder einem Salz davon behandelt werden.

Dabei zeigt sich, dass ein prophylaktischer Effekt bei Verabreichung der Verbindungen der Formel I schon mehrere Tage bis zu einigen, z.B. vier, Wochen vor der Infektion, und ein therapeutischer Effekt noch bei Verabreichung mehrere Tage, z.B. 1 Woche, nach der Infektion, eintritt.

Die Verbindungen der Formel I sind im obengenannten Test an der Maus bereits im Dosisbereich zwischen 0,001 mg/kg und 1 mg/kg wirksam.

Bemerkenswert ist auch das breite virale Spektrum, gegen das die obengenannten Verbindungen wirksam sind.

Die Verbindungen der Formel I können insbesondere zur Prophylaxe und Therapie von Krankheiten verwendet werden, die durch die nachstehend näher bezeichneten Viren hervorgerufen werden [zur Nomenklatur vgl. J.L.Melnick, Prog. med. Virol. 26, 214-232 (1980) und 28, 208-221 (1982)]:

DNA-Viren mit kubischer Symmetrie und nacktem Nukleokapsid, DNA-Viren mit umhülltem Virion sowie RNA-Viren mit kubischer und solche mit helikaler Symmetrie des Kapsids.

Bevorzugterweise verwendet man die Verbindungen der Formel I im Falle von DNA-Viren mit umhülltem Virion und kubischer Symmetrie des Kapsids, im Falle von RNA-Viren mit kubischer Symmetrie des Kapsids und nacktem Virion und im Falle von RNA-Viren mit helikaler Symmetrie des Kapsids, in denen die Nukleokapsidhülle bei der Oberflächenmembran gelegen ist, aber auch im Falle von Adenoviridae, Poxviridae und Coronaviridae, wie insbesondere menschlichen Coronaviren.

In erster Linie verwendet man die Verbindungen der Formel I im Falle von Herpesviridae, Picornaviridae und Myxoviren, aber auch im Falle von Mastadenoviren, wie insbesondere menschlichen Adenoviren, im Falle von Chordopoxvirinae, wie hauptsächlich Orthopoxviren, wie insbesondere z.B. Vacciniaviren, im Falle von Reoviridae, vornehmlich (insbesondere menschlichen) Rotaviren, sowie im Falle von Caliciviridae und Rhabdoviridae, wie in erster Linie Vesiculoviren des Menschen sowie von Pferden, Rindern und Schweinen.

Hauptsächlich verwendet man die Verbindungen der Formel I im Falle von Alphaherpesvirinae, wie Varicellaviren, z.B. menschlichen Varicella-Zoster Viren, Rhinoviren, Cardioviren und Orthomyxoviridae, aber auch im Falle von Betaherpesvirinae, wie insbesondere menschlichen Cytomegaloviren, im Falle von Aphthoviren, in erster Linie Apthoviren von Paarhufern, wie hauptsächlich von Rindern, sowie im Falle von Paramyxoviridae, wie in erster Linie Pneumoviren, z.B. respiratorischen Syncitialviren des Menschen, und wie daneben Morbilliviren oder Paramyxoviren, wie Parainfluenzaviren, z.B. menschlichen Parainfluenzaviren, einschliesslich der Sendaiviren sowie im Falle von Arboviren oder Vesiculoviren, z.B. Vesicular stomatitis Viren.

In allererster Linie verwendet man die Verbindungen der Formel I im Falle von Simplexviren, z.B. menschlichen Herpes simplex Viren der Typen 1 und 2, im Falle von menschlichen Encephalomyocarditisviren, im Falle von Influenzaviren, wie hauptsächlich Influenza A und Influenza B Viren, im Falle von Vaccinia und Parainfluenza Viren und ganz besonders im Falle der in den Beispielen genannten Viren.

Die Verbindungen der Formel I können zur Prophylaxe und Therapie von Virusinfektionen, insbesondere bei Warmblütern einschliesslich des Menschen, verwendet werden, indem man sie enteral oder parenteral, in erster Linie zusammen mit geeigneten Hilfs- oder Trägerstoffen, appliziert. Bevorzugterweise appliziert man

sie auf die Schleimhaut, z.B. intranasal, rektal, vaginal oder auf die Bindehaut des Auges, oder oral. Der antivirale Effekt tritt jedoch auch bei Applikation auf anderen Wegen, z.B. subkutan, intravenös, intramuskulär oder bei Applikation auf die normale Haut ein.

Die Dosierung des Wirkstoffes hängt u.a. von der Warmblüterspezies, der Abwehrlage des Organismus, der Applikationsweise und der Art des Virus ab. Die Dosis-Wirkungsbeziehung ist relaiiv schwach ausgeprägt.

Zur Vorbeugung appliziert man eine einmalige Dosis von etwa 0,01 mg bis etwa 25 mg, vorzugsweise 0,05 bis 7 mg, z.B. 0,5 mg, Wirkstoff an einen Warmblüter von etwa 70 kg Körpergewicht, z.B. den Menschen. Die prophylaktische Wirkung dieser Dosis erstreckt sich über mehrere Wochen. Bei Bedarft, z.B. in Zeiten erhöhter Ansteckungsgefahr, kann man die Verabreichung dieser Dosis wiederholen.

Die therapeutische Dosis für Warmblüter von etwa 70 kg Körpergewicht liegt zwischen 0,1 mg und 25 mg, vorzugsweise zwischen 1 und 10 mg, z.B. bei 5 mg, insbesondere bei oraler Applikation. Die Dosierung bei topischer, insbesondere intranasaler Applikation, liegt bis zum Faktor 10 niedriger. Bei Bedarf kann man die Verabreichung der Verbindungen der Formel I bis zum Eintritt einer Besserung der Erkrankung wiederholen. Oft genügt jedoch eine einmalige Applikation.

Somit betrifft die Erfindung auch eine Methode zur Prophylaxe oder Therapie von Virusinfektionen bei Warmblütern, welche die Verabreichung einer zur Prophylaxe oder Therapie von Virusinfektionen wirksamen Menge einer Verbindung der Formel I oder eines pharmazeutisch verwendbaren Salzes davon an einen Warmblüter umfasst.

Die Verbindungen der Formel I und ihre pharmazeutisch verwendbaren Salze hemmen die Wirkung von Phospholipase $A_2$ aus menschlichen Granulocyten ($IC_{50}$ zwischen etwa 0,1 μMol/Liter und 10 μMol/Liter). Phospholipase $A_2$ hemmt direkt die Freisetzung von Mediatoren entzündlicher Prozesse, z.B. von Arachidonsäure. Die erfindungsgemässen Verbindungen können daher auch als Antiallergika oder Antiinflammatorika verwendet werden.

Bevorzugt sind die obengenannten Verbindungen der Formel I, worin $R^3$ Wasserstoff und $R^4$ 1,2-Dihydroxy-ethyl, worin entweder nur die 2-Hydroxy-Gruppe oder beide Hydroxygruppen unabhängig voneinander mit einer $C_{10-24}$-Alkansäure oder einer $C_{18}$-Alkensäure verestert sind, bedeuten oder $R^3$ und $R^4$ je mit einer $C_{10-24}$-Alkansäure oder einer $C_{18}$-Alkensäure verestertes Hydroxymethyl bedeuten, und ihre Salze.

Bevorzugt sind insbesondere die obengenannten Verbindungen der Formel I, worin $R^3$ Wasserstoff und $R^4$ 1,2-Dihydroxyethyl, worin beide Hydroxygruppen unabhängig voneinander mit einer $C_{10-24}$-Alkansäure oder einer $C_{18}$-Alkensäure verestert sind, bedeuten oder $R^3$ und $R^4$ je mit einer $C_{10-24}$-Alkansäure oder einer $C_{18}$-Alkensäure veredtertes Hydroxymethyl bedeuten, und ihre Salze.

Sehr bevorzugt sind die obengenannten Verbindungen der Formel I, worin $R^3$ Wasserstoff und $R^4$ 1,2-Dihydroxyethyl, worin beide Hydroxygruppen unabhängig voneinander mit einer unverzweigten $C_{10-24}$-Alkansäure mit gerader Anzahl von C-Atomen oder einer unverzweigten $C_{18}$-Alkensäure mit 1-3 isolierten Doppelbindungen verestert sind, bedeuten oder $R^3$ und $R^4$ je mit einer unverzweigten $C_{10-24}$-Alkansäure mit gerader Anzahl von C-Atomen oder einer unverzweigten $C_{18}$-Alkensäure mit 1-3 isolierten Doppelbindungen verestertes Hydroxymethyl bedeuten, und ihre Salze.

Sehr bevorzugt sind auch die obengenannten Verbindungen der Formel I, worin Y unverzweigtes $C_1$-$C_5$-Alkylen oder den Rest $-CH_2-CH_2-CH_2-CO-NH-CH_2-$ bedeutet, und ihre Salze.

Sehr bevorzugt sind insbesondere die Verbindungen der Formel I, worin $R^1$ a) D-Aldohexosyl, welches in 4-Stellung glykosidisch mit D-Aldohexosyl verknüpft sein kann, b) 6-Desoxy-aldohexosyl oder c) 2-Acetylamino-2-desoxy-D-aldohexosyl, wobei in den oben unter a) bis c) genannten Resten vorhandene freie Hydroxygruppen peracetyliert sein können, bedeutet, X Sauerstoff oder Schwefel bedeutet, Y $C_{3-5}$-Alkylen, worin eine nicht endständige Methylengruppe durch Carbonylimino ersetzt sein kann, bedeutet, $R^2$ Wasserstoff oder Carboxy bedeutet, $R^3$ Wasserstoff und $R^4$ 1,2-Dihydroxy-ethyl, worin entweder nur die 2-Hydroxy-Gruppe oder beide Hydroxygruppen unabhängig voneinander mit einer unverzweigten $C_{10-24}$-Alkansäure oder einer unverzweigten $C_{18}$-Alkensäure verestert sind, bedeuten oder $R^3$ und $R^4$ je mit einer unverzweigten $C_{10-24}$-Alkansäure oder einer unverzweigten $C_{18}$-Alkensäure mit 1-3 isolierten Doppelbindungen verestertes Hydroxymethyl bedeuten, und ihre Salze.

Sehr bevorzugt sind insbesondere auch die Verbindungen der Formel I, worin $R^1$ a) D-Aldohexosyl, worin freie Hydroxygruppen peracetyliert sein können, b) D-Aldohexosyl, welches in 4-Stellung glykosidisch mit D-Aldohexosyl verknüpft ist, c) 6-Desoxy-aldohexosyl oder d) 2-Acetylamino-2-desoxy-D-aldohexosyl bedeutet, X Sauerstoff oder Schwefel bedeutet, Y Alkylen mit bis zu 5 C-Atomen, worin eine nicht endständige Methylengruppe durch Carbonylimino ersetzt sein kann, bedeutet, $R^2$ Wasserstoff oder Carboxy bedeutet, $R^3$ Wasserstoff und $R^4$ 1,2-Dihydroxy-ethyl, worin beide Hydroxygruppen mit einer unverzweigten $C_{10-20}$-Alkansäure mit gerader Anzahl von C-Atomen oder mit Oelsäure, verestert sind, bedeuten oder $R^3$ und $R^4$ je mit einer unverzweigten $C_{10-20}$-Alkansäure mit gerader Anzahl von C-Atomen oder mit Oelsäure verestertes Hydroxymethyl bedeuten, und ihre Salze.

Besonders bevorzugt sind die obengenannten Verbindungen der Formel I, worin $R^1$ D-Glucosyl, 2,3,4,6-Tetra-O-acetyl-D-glucosyl, D-Mannosyl, 2,3,4,6-Tetra-O-acetyl-D-mannosyl, D-Galactosyl, D-Cellobiosyl, D-Lactosyl, D-Maltosyl, L-Fucosyl oder 2-Acetylamino-2-desoxy-D-glucosyl bedeutet, und ihre Salze.

Besonders bevorzugt sind auch die Verbindungen der Formel I, worin $R^1$ 2,3,4,6-Tetra-O-acetyl-β-D-glucopyranosyl, β-D-Glucopyranosyl, 2,3,4,6-Tetra-O-acetyl-α-D-mannopyranosyl, β-D-Galactopyranosyl, β-D-Cellobiopyranosyl, β-D-Lactopyranosyl, β-D-Maltopyranosyl, β-L-Fucopyranosyl, 2-Acetylamino-2-desoxy-β-D-

glucopyranosyl oder α-D-Mannopyranosyl bedeutet,
X Sauerstoff oder Schwefel bedeutet,
Y Trimethylen, Methylen oder $-CH_2-CH_2-CH_2-CONH-CH_2-$ bedeutet,
$R^2$ Wasserstoff oder Carboxy bedeutet,
$R^3$ Wasserstoff und $R^4$ 1,2-Dilauroyloxy-ethyl, 1,2-Dimyristoyloxyethyl, 1,2-Dipalmitoyloxy-ethyl, 1,2-Distearoyloxy-ethyl, 1,2-n-Eicosanoyloxy-ethyl, 1,2-Dioleoyloxy-ethyl, n-Decanoyloxy-methyl oder 2-Palmitoyloxy-ethyl bedeuten oder $R^3$ und $R^4$ je Lauroyloxy-methyl bedeuten, und ihre pharmazeutisch verwendbaren Salze.

Ganz besonders bevorzugt sind die obengenannten Verbindungen der Formel I, worin $R^2$ Wasserstoff bedeutet und/oder worin Y Trimethylen oder den Rest $-CH_2-CH_2-CH_2-CO-NH-CH_2-$ bedeutet und/oder worin $R^3$ Wasserstoff und $R^4$ 1,2-Dipalmitoyloxy-ethyl oder $R^3$ und $R^4$ je Lauroyloxymethyl bedeuten, und ihre Salze.

In erster Linie bevorzugt sind die pharmazeutisch verwendbaren Salze der obengenannten Verbindungen der Formel I.

In allererster Linie bevorzugt sind die in den Beispielen beschriebenen Verbindungen der Formel I und/oder ihre pharmazeutisch verwendbaren Salze.

Die Verbindungen der Formel I werden in an sich bekannter Weise hergestellt, z.B. indem man

a) eine Verbindung der Formel II,

$R^1 - X^1$ (II)

worin $R^1$ die obengenannte Bedeutung hat und $X^1$ für die Gruppe X-H, worin X die obengenannte Bedeutung hat, oder für eine nucleophile Abgangsgruppe steht, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, oder ein reaktionsfähiges Derivat davon mit einer Verbindung der Formel III,

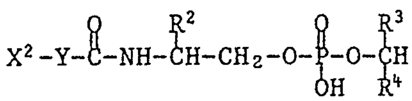

(III)

worin $X^2$ für die Gruppe H-X, worin X die obengenannte Bedeutung hat, oder für eine nucleophile Abgangsgruppe steht, und die übrigen Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, umsetzt und vorhandene Schutzgruppen abspaltet, oder

b) eine Carbonsäure der Formel IV,

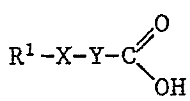

(IV)

worin die Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, oder ein reaktionsfähiges Carbonsäurederivat davon mit einer Aminoverbindung der Formel V,

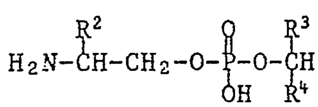

(V)

worin die Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, oder mit einem reaktionsfähigen Derivat davon umsetzt und vorhandene Schutzgruppen abspaltet, oder

c) zur Herstellung einer Verbindung der Formel I, worin Y für Alkylen steht, worin eine nicht-endständige Methylengruppe durch Carbonylimino ersetzt ist, eine Verbindung der Formel VI,

$R^1-X-Y^1-Z^1$ (VI)

worin $Y^1$ Alkylen und $Z^1$ Carboxy oder Amino bedeuten und die übrigen Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, oder ein reaktionsfähiges Derivat davon mit einer Verbindung der Formel VII,

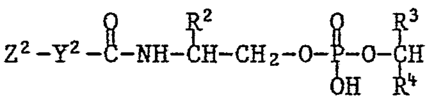

(VII)

worin $Z^2$ Amino bedeutet, wenn im Reaktionspartner der Formel VI $Z^1$ für Carboxy steht, oder worin $Z^2$ Carboxy bedeutet, wenn im Reaktionspartner der Formel VI $Z^1$ für Amino steht, und worin $Y^2$ Alkylen bedeutet, mit der Massgabe, dass der Rest $-Y^1-CONH-Y^2-$ bzw. $-Y^1-NHCO-Y^2-$ dem Rest Y entspricht, und die übrigen Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, oder mit einem reaktionsfähigen Derivat davon umsetzt und vorhandene Schutzgruppen abspaltet, oder

d) eine Verbindung der Formel VIII,

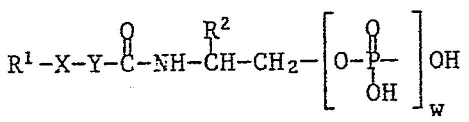

(VIII)

worin w für 0 oder 1 steht und die Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, oder ein reaktionsfähiges Derivat davon mit einer Verbindung der Formel IX,

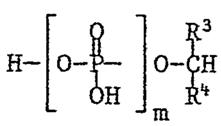

(IX)

worin m für 1 steht, wenn im Reaktionspartner der Formel VIII w für 0 steht, oder worin m für 0 steht, wenn im Reaktionspartner der Formel VIII w für 1 steht, und die Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, oder mit einem reaktionsfähigen Derivat davon umsetzt und vorhandene Schutzgruppen abspaltet, oder

e) eine Verbindung der Formel X,

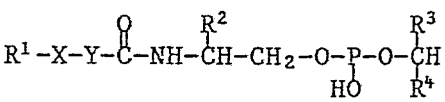

(X)

worin die Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, oder eine tautomere Verbindung mit einem geeigneten Oxidationsmittel oxidiert und vorhandene Schutzgruppen abspaltet, oder

f) eine Verbindung der Formel XI,

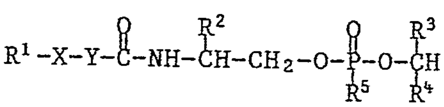

(XI)

worin $R^5$ für Halogen oder eine andere leicht hydrolysierbare Gruppe steht und die übrigen Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, hydrolysiert und vorhandene Schutzgruppen abspaltet, oder

g) eine Verbindung der Formel XII,

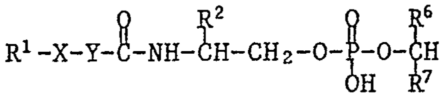

(XII)

worin $R^6$ Wasserstoff und $R^7$ 1,2-Dihydroxyethyl, 2-Hydroxy-ethyl, Hydroxymethyl oder 1,2-Dihydroxyethyl, worin eine der beiden Hydroxygruppen mit einer unsubstituierten aliphatischen $C_{2-24}$-Carbonsäure verestert ist, oder $R^6$ Hydroxymethyl und $R^7$ Hydroxymethyl oder Hydroxymethyl, das mit einer unsubstituierten aliphatischen $C_{10-24}$-Carbonsäure verestert ist, bedeuten, und die übrigen Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, mit einer unsubstituierten aliphatischen $C_{10-24}$-Carbonsäure oder einem reaktionsfähigen Säurederivat davon verestert und vorhandene Schutzgruppen abspaltet, oder

h) aus einer Verbindung der Formel XIII,

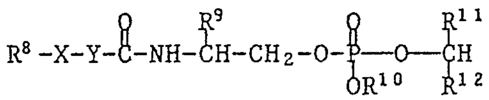

(XIII)

worin $R^8$ Aldohexosyl oder D-Aldohexosyl, welches in 4- oder 6-Stellung glykosidisch mit D-Aldohexosyl verknüpft ist, Aldopentosyl, 6-Desoxy-aldohexosyl oder 2-Acetylamino-2-desoxy-D-aldohexosyl, wobei in den obengenannten Resten mindestens eine Hydroxygruppe durch eine leicht abspaltbare Hydroxyschutzgruppe geschützt ist, bedeutet oder $R^8$ die Bedeutung von $R^1$ hat, X und Y die obengenannten Bedeutungen haben, $R^9$ durch eine leicht abspaltbare Schutzgruppe geschütztes Carboxy bedeutet oder die Bedeutung von $R^2$ hat, $R^{10}$ Wasserstoff oder eine leicht abspaltbare Phosphorsäureschutzgruppe bedeutet, $R^{11}$ Wasserstoff bedeutet und $R^{12}$ eine 1,2-Dihydroxy-ethylgruppe, worin eine Hydroxygruppe durch eine leicht abspaltbare Schutzgruppe geschützt ist und die andere Hydroxygruppe mit einer unsubstituierten aliphatischen $C_{10-24}$-Carbonsäure verestert ist, bedeutet oder $R^{12}$ die Bedeutung von $R^4$ hat, mit der Massgabe, dass in einer Verbindung der Formel XIII mindestens eine leicht abspaltbare Schutzgruppe vorhanden ist, die in dem gewünschten Endprodukt der Formel I nicht enthalten ist, diese Schutzgruppe(n) abspaltet, und nach Ausführung eines der obengenannten Verfahren a)-h) zur Herstellung eines Salzes erforderlichenfalls eine Verbindung der Formel I in eines ihrer Salze überführt.

Die obengenannten Verfahren werden im folgenden näher erläutert:

Bevorzugterweise stellt man die Verbindungen der Formel I nach den Verfahren b) oder c) her. Auch Verfahren h) ist leicht durchführbar. Leicht durchführbar sind auch die Verfahren e), f) und g), jedoch sind die hierfür benötigten Ausgangsstoffe nicht immer leicht zugänglich.

Verfahren a)

Eine nucleophile Abgangsgruppe $X^1$ oder $X^2$ ist insbesondere ein Halogenid mit einem Atomgewicht zwischen 35 und 127, d.h. ein Chlorid, Bromid oder Iodid. Ein reaktionsfähiges Derivat einer Verbindung der Formel II ist z.B. ein Isothiuroniumsalz, d.h. $X^1$ bedeutet $-S-C(-NH_2)=NH_2^{\oplus}$, wobei das Gegenion z.B. eines der obengenannten Halogenide ist.

Bevorzugterweise wird die Reaktion so durchgeführt, dass man eine Verbindung der Formel II, worin $X^1$ für eines der obengenannten Halogenide, z.B. Bromid oder Iodid, steht, mit einer Verbindung der Formel III oder einem geeigneten Salz, z.B. einem Silbersalz, davon, worin $X^2$ für die Gruppe HX steht, umsetzt. Verbindungen der Formel I, worin X für Schwefel steht, werden vorzugsweise so hergestellt, dass man ein Isothiuroniumsalz einer Verbindung der Formel II mit einer Verbindung der Formel III umsetzt, worin $X^2$ für eine nucleophile Abgangsgruppe, z.B. Iodid, steht.

Falls erforderlich, werden freie Hydroxygruppen im Rest $R^1$ und/oder $R^4$, freies Carboxy $R^2$ und/oder die Phosphorsäuregruppe durch leicht abspaltbare Schutzgruppen geschützt. Geeignete Schutzgruppen und ihre Abspaltung sind bei Verfahren b) beschrieben.

Die Ausgangsstoffe sind bekannt oder können nach an sich bekannten Verfahren z.B. analog geeigneter, in dieser Anmeldung beschriebener Verfahren, hergestellt werden.

Verfahren b)

Bevorzugterweise wird Verfahren b) so durchgeführt, dass man ein reaktionsfähiges Carbonsäurederivat einer Verbindung der Formel IV mit einer Verbindung der Formel V umsetzt, worin die Aminogruppe in freier Form vorliegt. Dabei erfolgt die Aktivierung der Carbonsäure IV vorzugsweise in situ, z.B. mit 1-Hydroxy-benzotriazol/Dicyclohexylcarbodiimid, weil dann auf den Schutz funktioneller Gruppen in den Verbindungen IV und V, vorausgesetzt, dass $R^2$ Wasserstoff bedeutet, verzichtet werden kann.

Gegebenenfalls werden freies Carboxy $R^2$ sowie erforderlichenfalls freies Hydroxy im Rest $R^1$ und $R^4$ sowie die Phosphorsäuregruppe durch leicht abspaltbare Schutzgruppen geschützt.

Schutzgruppen, ihre Einführung und Abspaltung sind beispielsweise beschrieben in "Protective Groups in Organic Chemistry", Plenum Press, London, New York 1973,und in "Methoden der organischen Chemie", Houben-Weyl, 4. Auflage, Bd. 15/1, Georg-Thieme-Verlag, Stuttgart 1974 sowie in Theodora W. Greene, "Protective Groups in Organic Synthesis, John Wiley & Sons, New York 1981. Charakteristisch für Schutzgruppen ist, dass sie leicht, d.h. ohne dass unerwünschte Nebenreaktionen stattfinden, z.B. solvolytisch, reduktiv, photolytisch oder auch unter physiologischen Bedingungen abspaltbar sind.

Hydroxyschutzgruppen sind z.B. Acylreste, wie gegebenenfalls, z.B. durch Halogen, substituiertes Niederalkanoyl, wie 2,2-Dichloracetyl, oder Acylreste von Kohlensäurehalbestern, insbesondere tert.-Butyloxycarbonyl, gegebenenfalls substituiertes Benzyloxycarbonyl, z.B. 4-Nitro-benzyloxycarbonyl, oder Diphenylmethoxycarbonyl, oder 2-Halogen-niederalkoxycarbonyl, wie 2,2,2-Trichlorethoxycarbonyl, ferner Trityl oder Formyl, oder organische Silyl- oder Stannylreste, ferner leicht abspaltbare verethernde Gruppen, wie tert.-Niederalkyl, z.B. tert.-Butyl, 2-oxa- oder 2-thia-aliphatische oder -cycloaliphatische Kohlenwasserstoffreste, in erster Linie 1-Niederalkoxyniederalkyl oder 1-Niederalkylthio-niederalkyl, z.B. Methoxy- methyl, 1-Methoxy-ethyl, 1-Ethoxy-ethyl, Methylthiomethyl, 1-Methylthioethyl oder 1-Ethylthioethyl, oder 2-Oxa- oder 2-Thiacycloalkyl mit 5-6 Ringatomen, z.B. Tetrahydrofuryl oder 2-Tetrahydropyranyl oder entsprechende Thiaanaloge, sowie gegebenenfalls substituiertes 1-Phenylniederalkyl, wie gegebenenfalls substituiertes Benzyl oder Diphenylmethyl, wobei als Substituenten der Phenylreste z.B. Halogen, wie Chlor, Niederalkoxy, wie Methoxy und/oder Nitro in Frage kommen.

Carboxylgruppen sind üblicherweise in veresterter Form geschützt, wobei solche Estergruppierungen unter schonenden Bedingungen leicht spaltbar sind. In dieser Art geschützte Carboxylgruppen enthalten als veresternde Gruppen in erster Linie in 1-Stellung verzweigte oder in 1- oder 2-Stellung geeignet substituierte Niederalkylgruppen. Bevorzugte, in veresterter Form vorliegende Carboxylgruppen sind u.a. tert.-Niederalkoxycarbonyl, z.B. tert.-Butyloxycarbonyl, Arylmethoxycarbonyl mit einem oder zwei Arylresten, wobei diese gegebenenfalls z.B. durch Niederalkyl, wie tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellen, wie gegebenenfalls, z.B. wie oben erwähnt, substituiertes Benzyloxycarbonyl, z.B. 4-Methoxybenzyloxycarbonyl, oder 4-Nitrobenzyloxycarbonyl, oder gegebenenfalls, z.B. wie oben erwähnt, substituiertes Diphenylmethoxycarbonyl, z.B. Diphenylmethoxycarbonyl oder Di-(4-methoxyphenyl)-methoxycarbonyl, 1-Niederalkoxyniederalkoxycarbonyl, wie Methoxymethoxycarbonyl, 1-Methoxyethoxycarbonyl oder 1-Ethoxymethoxycarbonyl, 1-Niederalkylthioniederalkoxycarbonyl, wie 1-Methylthiomethoxycarbonyl oder 1-Ethylthioethoxycarbonyl, Aroylmethoxycarbonyl, worin die Aroylgruppe gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichlorethoxycarbonyl, 2-Bromethoxycarbonyl oder 2-Iodethoxycarbonyl, oder 2-(trisubstituiertes Silyl)-ethoxycarbonyl, worin die Substituenten unabhängig voneinander je einen gegebenenfalls substituierten, z.B. durch Niederalkyl, Niederalkoxy, Aryl, Halogen, und/oder Nitro substituierten, aliphatischen, araliphatischen, cycloaliphatischen oder aromatischen Kohlenwasserstoffrest, wie entsprechendes, gegebenenfalls substituiertes Niederalkyl, Phenylniederalkyl, Cycloalkyl oder Phenyl, bedeuten, z.B. 2-Triniederalkylsilylethoxycarbonyl, 2-Trimethylsilylethoxycarbonyl oder 2-(Di-n-butyl-methyl-silyl)-ethoxycarbonyl, oder 2-Triarylsilylethoxycarbonyl, wie 2-Triphenylsilylethoxycarbonyl.

Die oben und im folgenden erwähnten organischen Silyl- oder Stannylreste enthalten vorzugsweise Niederalkyl, insbesondere Methyl, als Substituenten der Silizium- oder Zinn-Atome. Entsprechende Silyl-oder Stannylgruppen sind in erster Linie Triniederalkylsilyl, insbesondere Trimethylsilyl, ferner Dimethyl-tert.-butylsilyl, oder entsprechend substituiertes Stannyl, z.B. Tri-n-butylstannyl.

Bevorzugte geschützte Carboxylgruppen sind tert.-Niederalkoxycarbonyl, wie tert.-Butoxycarbonyl, und in erster Linie gegebenenfalls, z.B. wie oben erwähnt, substituiertes Benzyloxycarbonyl, wie 4-Nitrobenzyloxycarbonyl, oder Diphenylmethoxycarbonyl, vor allem 2-(Trimethylsilyl)-ethoxycarbonyl.

Die Phosphorsäuregruppe ist z.B. in Form des Methyl-, Phenyl-, 2-Chlor-phenyl-, 2,5-Dichlor-phenyl-, 4-Nitro-phenyl-, Benzyl-, 2-(4-Nitro-phenyl)-ethyl- oder 2-Cyan-ethyl-esters geschützt.

Reaktionsfähige Carbonsäurederivate sind in erster Linie reaktionsfähige aktivierte Ester oder reaktionsfähige Anhydride, ferner reaktionsfähige cyclische Amide.

Aktivierte Ester von Säuren sind insbesondere am Verknüpfungskohlenstoffatom des veresternden Restes ungesättigte Ester, z.B. vom Vinylester-Typ, wie eigentliche Vinylester (die man z.B. durch Umesterung eines entsprechenden Esters mit Vinylacetat erhalten kann; Methode des aktivierten Vinylesters), Carbamoylvinylester (die man z.B. durch Behandeln der entsprechenden Säure mit einem Isoxazoliumreagens erhalten kann; 1,2-Oxazolium- oder Woodward-Methode), oder 1-Niederalkoxyvinylester (die man z.B. durch Be handeln der entsprechenden Säure mit einem Niederalkoxyacetylen erhalten kann; Ethoxyacetylen-Methode), oder Ester vom Amidinotyp, wie N,N'-disubstituierte Amidinoester (die man z.B. durch Behandeln der entsprechenden Säure mit einem geeigneten N,N'-disubstituierten Carbodiimid, z.B. N,N'-Dicyclohexylcarbodiimid, erhalten kann; Carbodiimid-Methode), oder N,N-disubstituierte Amidinoester (die man z.B. durch Behandeln der entsprechenden Säure mit einem N,N-disubstituierten Cyanamid erhalten kann; Cyanamid-Methode), geeignete Arylester, insbesondere durch Elektronen-anziehende Substituenten geeignet substituierte Phenylester (die man z.B. durch Behandeln der entsprechenden Säure mit einem geeignet substituierten Phenol, z.B. 4-Nitrophenol, 4-Methylsulfonyl-phenol, 2,4,5-Trichlorphenol, 2,3,4,5,6-Pentachlor-phenol oder 4-Phenyldiazophenol, in Gegenwart eines Kondensationsmittels, wie N,N'-Dicyclohexyl-carbodiimid, erhalten kann; Methode der aktivierten Arylester), Cyanmethylester (die man z.B. durch Behandeln der entsprechenden Säure mit Chloracetonitril in Gegenwart einer Base erhalten kann; Cyanmethylester-Methode), Thioester, insbesondere gegebenenfalls, z.B. duch Nitro, substituierte Phenyl-thioester (die man z.B. durch Behandeln der entsprechenden Säure mit gegebenenfalls, z. B. durch Nitro, substituierten Thiophenolen, u.a. mit Hilfe der Anhydrid-oder Carbodiimid-Methode, erhalten kann; Methode der aktivierten Thiolester) oder Amino- oder Amidoester (die man z.B. durch Behandeln der entsprechenden Säure mit einer N-Hydroxy-amino- bzw. N-Hydroxy-amido-Verbindung, z.B. N-Hydroxy-succinimid, N-Hydroxy-piperidin, N-Hydroxyphthalimid oder 1-Hydroxy-benztriazol, z.B. nach der Anhydrid- oder Carbodiimid-Methode, erhalten kann; Methode der aktivierten N-Hydroxyester).

Anhydride von Säuren können symmetrische oder vorzugsweise gemischte Anhydride dieser Säuren sein, so z.B. Anhydride mit anorganischen Säuren, wie Säurehalogenide, insbesondere Säurechloride (die man z.B. durch Behandeln der entsprechenden Säure mit Thionylchlorid, Phosphorpentachlorid oder Oxalylchlorid erhalten kann; Säurechloridmethode), Azide (die man z.B. aus einem entsprechenden Säureester über das entsprechende Hydrazid und dessen Behandlung mit sal petriger Säure erhalten kann; Azidmethode), Anhydride mit Kohlensäurehalbderivaten, wie mit entsprechenden Estern, z.B. Kohlensäureniederalkylhalbestern (die man z.B. durch Behandeln der entsprechenden Säure mit Halogen-, wie Chlorameisensäure-niederalkylestern oder mit einem 1-Niederalkoxycarbonyl-2-niederalkoxy-1,2-dihydro-chinolin, z.B. 1-Niederalkoxycarbonyl-2-ethoxy-1,2-dihydrochinolin, erhalten kann; Methode der gemischten 0-Alkyl-kohlensäureanhydride), oder Anhydride mit dihalogenierter, insbesondere dichlorierter Phosphorsäure (die man z.B. durch Behandeln der entsprechenden Säure mit Phosphoroxychlorid erhalten kann; Phosphoroxychloridmethode), oder Anhydride mit organischen Säuren, wie gemischte Anhydride mit organischen Carbonsäuren (die man z.B. durch Behandeln der entsprechenden Säure mit einem gegebenenfalls substituierten Niederalkan- oder Phenylalkancarbonsäurehalogenid, z.B. Phenylessigsäure-, Pivalinsäure- oder Trifluoressigsäure-chlorid erhalten kann; Methode der gemischten Carbonsäureanhydride) oder mit organischen Sulfonsäuren (die man z.B. durch Behandeln eines Salzes, wie eines Alkalimetallsalzes, der entsprechenden Säure, mit einem geeigneten organischen Sulfonsäurehalogenid, wie Niederalkan- oder Aryl-, z.B. Methan- oder p-Toluolsulfonsäurechlorid, erhalten kann; Methode der gemischten Sulfonsäureanhydride), sowie symmetrische Anhydride (die man z.B. durch Kondensation der entsprechenden Säure in Gegenwart eines Carbodiimids oder von 1-Diethylaminopropin erhalten kann; Methode der symmetrischen Anhydride).

Geeignete cyclische Amide sind insbesondere Amide mit fünfgliedrigen Diazacyclen aromatischen Charakters, wie Amide mit Imidazolen, z.B. Imidazol (die man z.B. durch Behandeln der entsprechenden Säure mit N,N′-Carbonyldiimidazol erhalten kann; Imidazolid-Methode), oder Pyrazolen, z.B. 3,5-Dimethyl-pyrazol (die man z.B. über das Säurehydrazid durch Behandeln mit Acetylaceton erhalten kann; Pyrazolid-Methode).

Wie erwähnt können Derivate von Säuren, die als Acylierungsmittel verwendet werden, auch in situ gebildet werden. So kann man z.B. N,N′-disubstituierte Amidinoester in situ bilden, indem man das Gemisch der Aminoverbindung und der als Acylierungsmittel verwendeten Säure in Gegenwart eines geeigneten N,N-disubstituierten Carbodiimids, z.B. N,N′-Dicyclohexylcarbodiimid, zur Reaktion bringt. Ferner kann man Amino- oder Amidoester der als Acylierungsmittel verwendeten Säuren in Gegenwart des zu acylierenden Ausgangsmaterials bilden, indem man das Gemisch der entsprechenden Säure-und Amino-Ausgangsstoffe in Gegenwart eines N,N′-disubstituierten Carbodiimids, z.B. N,N′-Dicyclohexyl-carbodimid, und eines N-Hydroxy-amins oder N-Hydroxy-amids, z.B. N-Hydroxysuccinimid, gegebenenfalls in Anwesenheit einer geeigneten Base, z.B. 4-Dimethylamino-pyridin, umsetzt.

Die Reaktion kann in an sich bekannter Weise durchgeführt werden, wobei die Reaktionsbedingungen in erster Linie davon abhängen, ob und wie die Carboxylgruppe des Acylierungsmittels aktiviert ist, üblicherweise in Gegenwart eines geeigneten Lösungs- oder Verdünnungsmittels oder eines Gemisches von solchen, und, falls notwendig, in Gegenwart eines Kondensationsmittels, das z.B., wenn die an der Reaktion teilnehmende Carboxylgruppe als Anhydrid vorliegt, auch ein Säure-bindendes Mittel sein kann, unter Kühlen oder Erwärmen, z.B. in einem Temperaturbereich von etwa -30°C bis etwa +150°C, insbesondere von etwa 0°C bis +100°C, vorzugsweise von Raumtemperatur (ca. +20°C) bis +70°C, hauptsächlich zwischen Raumtemperatur und +40°C, in einem geschlossenen Reaktionsgefäss und/oder in der Atmosphäre eines Inertgases, z.B. Stickstoff. Uebliche Kondensationsmittel sind z.B. Carbodiimide, beispielsweise N,N′-Diethyl-, N,N′-Dipropyl-, N,N′-Dicyclohexyl- oder N-Ethyl-N′(3-dimethylaminopropyl)-carbodiimid, geeignete Carbonylverbindungen, beispielsweise Carbonyldiimidazol, oder 1,2-Oxazoliumverbindungen, z.B. 2-Ethyl-5-phenyl-1,2-oxazolium-3′-sulfonat und 2-tert.-Butyl-5-methylisoxazolium-perchlorat, oder eine geeignete Acylaminoverbindung, z.B. 2-Ethoxy-1-ethoxycarbonyl-1,2-dihydrochinolin. Uebliche säurebindende Kondensationsmittel sind z.B. Alkalimetallcarbonate oder -hydrogencarbonate, z.B. Natrium- oder Kaliumcarbonat oder -hydrogencarbonat (üblicherweise zusammen mit einem Sulfat), oder organische Basen, wie üblicherweise sterisch gehinderte Triniederalkylamine, z.B. N,N-Diisopropyl-N-ethyl-amin.

Die Abspaltung der Schutzgruppen, z.B. der Carboxyl- oder Hydroxyschutzgruppen, die nicht Bestandteil des gewünschten Endprodukts der Formel I sind, erfolgt in an sich bekannter Weise, z.B. mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig, wobei auch enzymatische Methoden verwendet werden können.

So kann man tert.-Niederalkoxycarbonyl oder in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B. durch Behandeln mit einer geeigneten Säure, wie Ameisensäure

oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, freigesetzt werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlor-mandelsäure oder Weinsäure, oder eines Alkohols oder Thiols, wobei man vorzugsweise Wasser zugibt, in freies Carboxyl überführen. Durch Behandeln mit einem reduzierenden Metall oder Metallsalz, wie oben beschrieben, kann man auch 2-Halogen-niederalkoxycarbonyl (gegebenenfalls nach Umwandlung einer 2-Brom-niederalkoxycarbonylgruppe in eine entsprechende 2-Iod-niederalkoxycarbonylgruppe) oder Aroylmethoxycarbonyl in freies Carboxyl umwandeln, wobei Aroylmethoxycarbonyl ebenfalls durch Behandeln mit einem nucleophilen, vorzugsweise salzbildenden Reagens, wie Natriumthiophenolat oder Natriumiodid, gespalten werden kann. Substituiertes 2-Silylethoxycarbonyl kann auch durch Behandeln mit einem, das Fluoridanion liefernden Salz der Fluorwaserstoffsäure, wie einem Alkalimetallfluorid, z.B. Natrium- oder Kaliumfluorid, in Anwesenheit eines macrocyclischen Polyethers ("Kronenether"), oder mit einem Fluorid einer organischen quaternären Base, wie Tetra-niederalkylammoniumfluorid oder Triniederalkyl-aryl-ammoniumfluorid, z.B. Tetraethylammoniumfluorid oder Tetrabutylammoniumfluorid, in Gegenwart eines aprotischen polaren Lösungsmittels, wie Dimethylsulfoxid oder N,N-Dimethylacetamid, in freies Carboxyl übergeführt werden.

Eine durch eine geeignete Acylgruppe, eine organische Silylgruppe oder durch gegebenenfalls substituiertes 1-Phenylniederalkyl geschützte Hydroxygruppe wird je nach Art der Schutzgruppe solvolytisch oder reduktiv freigesetzt. Durch gegebenenfalls substituiertes 1-Phenylniederalkyl, z.B. Benzyl, geschütztes Hydroxy wird vorzugsweise durch katalytische Hydrierung, z.B. in Gegenwart eine Palladium-auf-Kohle-Katalysators freigesetzt. Eine durch 2,2-Dichloracetyl geschützte Hydroxygruppe wird z.B. durch basische Hydrolyse, eine durch tert.-Niederalkyl oder durch einen 2-oxa- oder 2-thia-aliphatischen oder -cycloaliphatischen Kohlenwasserstoffrest veretherte Hydroxygruppe durch Acidolyse, z.B. durch Behandeln mit einer Mineralsäure oder einer starken Carbonsäure, z.B. Trifluoressigsäure, freigesetzt. Zwei Hydroxygruppen, die zusammen mittels einer vorzugsweise substituierten Methylengruppe, wie durch Niederalkyliden, z.B. Isopropyliden, Cycloalkyliden, z.B. Cyclohexyliden, oder Benzyliden, geschützt sind, können durch saure Solvolyse, besonders in Gegenwart einer Mineralsäure oder einer starken organischen Säure, freigesetzt werden. Mit einem organischen Silylrest, z.B. Trimethylsilyl, verethertes Hydroxy kann auch mit einem Fluoridanionen liefernden Salz der Fluorwasserstoffsäure, z.B. Tetrabutylammoniumfluorid, freigesetzt werden.

Eine in Form des Methylesters geschützte Phosphorsäuregruppe kann z.B. mit einem geeigneten Lithiumhalogenid, vorzugsweise Lithiumbromid, in einem Lösungsmittel, wie z.B. Aceton, freigesetzt werden. Eine in Form des Benzylesters geschützte Phosphorsäuregruppe kann z.B. hydrogenolytisch in Gegenwart von Palladium/Kohlenstoff-Katalysatoren freigesetzt werden. Phenylester werden mit Wasserstoff in Gegenwart von Platin- oder gemischten Platin-Palladium-Katalysatoren gespalten. Eine in Form des 2-Chlor-phenyl-, 2,5-Dichlor-phenyl-oder 4-Nitro-phenylesters geschützte Phosphorsäuregruppe wird durch Oximat-Spaltung, z.B. mittels des N,N'-Tetramethylguanidiniumsalzes von 2-Nitro-benzaldoxim, 4-Nitro-benzaldoxim oder 2-Pyridin-aldoxim, freigesetzt. Die 2-(4-Nitro-phenyl)-ethyl-Schutzgruppe wird durch β-Eliminierung mittels DBU (1,8-Diazabicyclo[5.4.0]undec-7-en) in einem aprotischen Lösungsmittel und die 2-Cyan-ethyl-Schutzgruppe durch β-Eliminierung mittels Triethylamin/Wasser abgespalten.

Bevorzugt werden beim Vorhandensein von mehreren geschützten funktionellen Gruppen die Schutzgruppen so gewählt, dass gleichzeitig mehr als eine solche Gruppe abgespalten werden kann, beispielsweise acidolytisch, wie durch Behandeln mit Trifluoressigsäure oder Ameisensäure, oder reduktiv, wie durch Behandeln mit Zink und Essigsäure, oder mit Wasserstoff und einem Hydrierkatalysator, wie einem Palladium-Kohle-Katalysator. Wenn die gewünschten Endstoffe Schutzgruppen enthalten, z.B. wenn $R^2$ Benzyloxycarbonyl bedeutet, werden diejenigen Schutzgruppen, die nach erfolgter Reaktion abgespalten werden sollen, so gewählt, dass sie regioselektiv wieder abgespalten werden können, z.B. kann mit einem organischen Silylrest verethertes Hydroxy im Rest $R^1$ oder $R^4$ mit Fluorid freigesetzt werden, wobei eine Benzyl-Schutzgruppe im Rest $R^2$ erhalten bleibt.

Verfahren c)

Verfahren c) wird analog Verfahren b) durchgeführt.

Verfahren d)

Vorzugsweise setzt man eine Verbindung der Formel VIII, worin w für 0 steht, mit einer Verbindung der Formel IX, worin m für 1 steht, oder insbesondere mit einem reaktionsfähigen Derivat einer solchen Verbindung der Formel IX um.

Ein reaktionsfähiges Derivat einer Verbindung der Formel VIII, worin w für 1 steht, oder einer Verbindung der Formel IX, worin m für 1 steht, ist z.B. ein Mono- oder Bisanhydrid mit einer starken Säure, insbesondere einer

Mineralsäure, wie vornehmlich einer Halogenwasserstoffsäure, wie hauptsächlich Chlorwasserstoffsäure. Die zweite saure Phosphorsäuregruppe kann als solche, als Anhydrid wie oben geschildert oder in geschützter Form vorliegen. Reaktionsfähige Phosphorsäurederivate sind z.B. auch Benzo-1,2,3-triazol-1-yl-ester, N-Succinimidyl-ester und 1,2,4-Triazolide.

Die Bildung reaktionsfähiger Phosphorsäurederivate kann auch in situ in Gegenwart von Verbindungen erfolgen, die mit Phosphorsäure oder deren Monoestern zumindest intermediär reaktionsfähige Verbindungen mit anhydrid- oder enolesterartigem Charakter einzugehen vermögen, z.B. in Gegenwart von p-Toluolsulfonsäurechlorid, Cyanurchlorid, N-Alkyl-5-phenylisoxazoliumsalzen, Ethoxyacetylen oder bevorzugterweise Trichloracetonitril oder insbesondere einem Carbodiimid, wie hauptsächlich Dicyclohexylcarbodiimid. Beispielsweise kann man einen Phosphorsäuremonoester der Formeln VIII oder IX, worin w bzw. m für 1 stehen, mit überschüssigem Alkohol der Formeln IX bzw. VIII, worin m bzw. w für 0 stehen, in Gegenwart der mehrfachen, z.B. fünffachen, molaren Menge von Dicyclohexylcarbodiimid in An- oder Abwesenheit eines tertiären Amins umsetzen.

Wenn in einem Phosphorsäuremonoester beide sauren Gruppen als Anhydrid mit einer Halogenwasserstoffsäure vorliegen, kann man zunächst neben dem Triester auch Phosphorsäurediester-halogenide erhalten, die anschliessend durch Wasser, wasserabgebende Mittel oder durch Erhitzen mit tertiären Alkoholen, wie tert.-Butanol oder Tetrahydropyranol, zu Diestern hydrolysiert werden müssen [siehe Verfahren f)].

Wenn man von einem Phosphorsäuremonoester-dihalogenid, z.B. einem Phosphorsäuremonoester-dichlorid, ausgeht, führt man die Umsetzung bevorzugterweise in Anwesenheit eines tertiären Amins, wie Pyridin, Lutidin oder Chinolin durch, wobei eine zusätzliche Aktivierung des Esterchlorids durch Dimethylformamid bewirkt wird.

Eine bevorzugte Ausführungsform des Verfahrens d) ist die Umsetzung eines Phosphorsäuremonoester-dichlorids mit dem entsprechenden Alkohol in Gegenwart eines tertiären Amins, gefolgt von der Hydrolyse des zunächst erhaltenen Phosphorsäurediesterhalogenids.

In einem reaktionsfähigen Derivat einer Verbindung der Formeln VIII oder IX, worin w bzw. m für 0 stehen, liegt die an der Reaktion teilnehmende Hydroxygruppe in reaktionsfähiger, veresterter Form vor.

Reaktionsfähiges verestertes Hydroxy ist z.B. mit einer starken anorganischen oder organischen Säure verestertes Hydroxy, wie mit einer Mineralsäure, z.B. Halogenwasserstoffsäure, wie Chlorwasserstoff-, Bromwasserstoff- oder Iodwasserstoffsäure, ferner Schwefelsäure, oder Halogenschwefelsäure, z.B. Fluorschwefelsäure, oder mit einer starken organischen Sulfonsäure, wie einer gegebenenfalls, z.B. durch Halogen, wie Fluor, substituierten Niederalkansulfonsäure oder einer aromatischen Sulfonsäure, z.B. einer gegebenenfalls durch Niederalkyl, wie Methyl, Halogen, wie Brom, und/oder Nitro substituierten Benzolsulfonsäure, z.B. einer Methansulfon-, Trifluormethansulfon- oder p-Toluolsulfonsäure verestertes Hydroxy, bevorzugterweise ein Chlorid, Bromid oder Iodid.

Die Reaktion kann so durchgeführt werden, dass man ein reaktionsfähiges Phosphorsäurederivat der Formeln VIII oder IX mit einem Alkohol der Formeln IX bzw. VIII in unaktivierter Form umsetzt, oder indem man einen reaktionsfähigen veresterten Alkohol der Formeln VIII oder IX mit einem Phosphorsäurederivat der Formeln IX bzw. VIII in unaktivierter Form oder einem reaktionsfähigen Salz davon umsetzt.

Als Salze von Verbindungen der Formeln VIII oder IX verwendet man im Hinblick auf die beabsichtigte nucleophile Substitutionsreaktion besonders reaktionsfähige Salze, z.B. Salze, wie Silbersalze, die mit der nucleophilen Abgangsgruppe im Reaktionspartner, z.B. einem der obengenannten Halogenidionen, einen schwerlöslichen Niederschlag zu bilden vermögen, oder Salze mit grossem Kation, z.B. Cäsiumsalze, in denen die Nucleophilie des Phosphatrestes erhöht ist. Zur Erhöhung der Nucleophilie des Phosphatrestes kann das Gegenion auch räumlich entfernt werden, z.B. durch Zugabe von Komplexbildnern, wie Kronenethern, z.B. 18-Krone-6. Bei Verwendung von 18-Krone-6 kann man die Reaktion mit einem Kaliumsalz durchführen.

Eine bevorzugte Ausführungsform des Verfahrens d) ist die Umsetzung des Silbersalzes eines Phosphorsäuremonoesters der Formeln VIII oder IX, worin eine der beiden sauren Gruppen durch eine leicht abspaltbare Schutzgruppe geschützt ist, mit einem reaktionsfähigen Alkohol der Formel IX bzw. VIII, worin die OH-Gruppe durch Chlor, Brom oder vorzugsweise Iod ersetzt ist.

Geeignete Schutzgruppen und ihre Abspaltung sind bei Verfahren b) beschrieben.

Verfahren e)

Die Verbindungen der Formel X liegen zum überwiegenden Teil in der tautomeren Form vor, worin ein Proton direkt an Phosphor gebunden ist. Die Oxidation kann z.B. mit wässerigem Kaliumpermanganat bei Temperaturen um 0°C durchgeführt werden. In wässerigem Medium sind auch Alkalijodate, -perjodate und -hypochlorite, Peressigsäure, N-Chlor-4-methyl-benzol-sulfonsäureamid u.a. als Oxidationsmittel geeignet.

Geeignete Schutzgruppen und ihre Abspaltung sind bei Verfahren b) beschrieben.

Verfahren f)

Halogen $R^5$ ist Brom oder Iod, in allererster Linie aber Chlor.

Andere leicht hydrolysierbare Gruppen als Halogen sind z.B. Benzo-1,2,3-triazol-1-yloxy, N-Succinimidyloxy und 1,2,4-Triazol-1-yl.

Die Hydrolyse wird mit Wasser oder einem wasserabgebenden Mittel, vorzugsweise bei erhöhter Temperatur, z.B. zwischen 30 und 95°C, durchgeführt.

Die Ausgangsstoffe sind z.B. wie bei Verfahren d) beschrieben oder durch Chlorierung der entsprechenden

Phosphorigsäurediester, z.B. mit elementarem Chlor, erhältlich.

Geeignete Schutzgruppen und ihre Abspaltung sind bei Verfahren b) beschrieben.

Verfahren g)

In einer Verbindung der Formel XII müssen freie Hydroxygruppen im Rest $R^1$ geschützt werden. Wenn erwünscht, kann auch freies Carboxy $R^2$ und/oder die Phosphorsäuregruppe geschützt werden. Geeignete Schutzgruppen und ihre Abspaltung sind bei Verfahren b) beschrieben.

Reaktionsfähige Derviate der unsubstituierten aliphatischen Carbonsäure sind analog den bei Verfahren b) beschriebenen, wobei die Aktivierung auch in situ, d.h. in Gegenwart der Verbindung XIII erfolgen kann.

Die Reaktion wird analog Verfahren b) durchgeführt.

Das Ausgangsmaterial der Formel XII ist z.B. analog Verfahren b) erhältlich, wenn man anstelle von Verbindungen der Formel V Verbindungen verwendet, worin mindestens eine Hydroxygruppe in den Resten $R^3$ und $R^4$ durch eine leicht abspaltbare Schutzgruppe geschützt ist, und diese Schutzgruppen nach erfolgter Reaktion mit der Carbonsäure IV abspaltet. Alternativ kann man eine Ausgangsverbindung der Formel XII, worin $R^3$ Wasserstoff und $R^4$ 1,2-Dihydroxy-ethyl, worin die 2-Hydroxygruppe mit einer unsubstituierten aliphatischen $C_{10-24}$-Carbonsäure verestert ist, bedeuten, auch herstellen, indem man eine Verbindung der Formel I, worin $R^3$ Wasserstoff und $R^4$ 1,2-Dihydroxy-ethyl, worin die 2-Hydroxygruppe mit einer unsubstituierten aliphatischen $C_{10-24}$-Carbonsäure verestert ist und die 1-Hydroxygruppe mit einer unsubstituierten aliphatischen $C_{2-24}$-Carbonsäure verestert ist, bedeuten, mit einem zur regioselektiven Abspaltung des die 1-Hydroxygruppe veresternden Restes geeigneten Enzym umsetzt. Ein geeignetes Enzym ist z.B. Phospholipase $A_2$, das im Handel erhältlich ist (z.B. Boehringer AG, Mannheim, Bundesrepublik Deutschland).

Verfahren h)

Geeignete Schutzgruppen und ihre Abspaltung sind bei Verfahren b) beschrieben.

Zusatzoperationen

Zur Herstellung einer Verbindung der Formel I, worin $R^2$ Niederalkoxycarbonyl, Benzyloxycarbonyl oder Carbamoyl bedeutet, kann eine Verbindung der Formel I, worin $R^2$ Carboxy bedeutet, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, oder ein reaktionsfähiges Säurederivat davon verestert oder amidiert werden. Dabei kann die Veresterung oder Amidierung so durchgeführt werden, dass man ein reaktionsfähiges Säurederivat mit dem entsprechenden Niederalkanol oder mit Benzylalkohol oder Ammoniak umsetzt. Die reaktionsfähigen Säurederivate sind analog den bei Verfahren b) beschriebenen. Vorzugsweise wird aber die Veresterung so durchgeführt, dass man eine Verbindung der Formel I, worin $R^2$ nicht-aktiviertes Carboxy bedeutet, mit einem reaktionsfähigen Veresterungsmittel umsetzt.

Geeignete Mittel zur Veresterung sind beispielsweise entsprechende Diazoverbindungen, wie gegebenenfalls substituierte Diazoniederalkane, z.B. Diazomethan, Diazoethan oder Diazo-n-butan. Diese Reagenzien werden in Gegenwart eines geeigneten inerten Lösungsmittels, wie eines aliphatischen, cycloaliphatischen oder aromatischen Kohlenwasserstoffs, wie Hexan, Cyclohexan, Benzol oder Toluol, eines halogenierten aliphatischen Kohlenwasserstoffs, z.B. Methylenchlorid, oder eines Ethers, wie eines Diniederalkylethers, z.B. Diethylether, oder eines cyclischen Ethers, z.B. Tetrahydrofuran oder Dioxan, oder eines Lösungsmittelgemisches, und, je nach Diazoreagens, unter Kühlen, bei Raumtemperatur oder unter leichtem Erwärmen, ferner, wenn notwendig, in einem geschlossenen Gefäss und/oder unter einer Inertgas-, z.B. Stickstoffatmosphäre, zur Anwendung gebracht.

Weitere geeignete Mittel zur Veresterung sind Ester entsprechender Alkohole, in erster Linie solche mit starken anorganischen oder organischen Säuren, wie Mineralsäuren, z.B. Halogenwasserstoffsäuren, wie Chlorwasserstoff-, Bromwasserstoff- oder Iodwasserstoffsäure, ferner Schwefelsäure, oder Halogen-schwefelsäure, z.B. Fluorschwefelsäure, oder starken organischen Sulfonsäuren, wie gegebenenfalls, z.B. durch Halogen, wie Fluor, substituierten Niederalkansulfonsäuren, oder aromatischen Sulfonsäuren, wie z.B. gegebenenfalls durch Niederalkyl, wie Methyl, Halogen, wie Brom, und/oder Nitro substituierten Benzolsulfonsäuren, z.B. Methansulfon-, Trifluormethansulfon- oder p-Toluolsulfonsäure. Solche Ester sind u.a. Niederalkylhalogenide, Diniederalkylsulfate, wie Dimethylsulfat, ferner Fluorsulfonsäureester, wie -niederalkylester, z.B. Fluorsulfonsäuremethylester, oder gegebenenfalls Halogen-substituierte Methansulfonsäure-niederalkylester, z.B. Trifluormethansulfonsäuremethylester. Sie werden üblicherweise in Gegenwart eines inerten Lösungsmittels, wie eines gegebenenfalls halogenierten, wie chlorierten, aliphatischen, cycloaliphatischen oder aromatischen Kohlenwasserstoffs, z.B. Methylenchlorid, eines Ethers, wie Dioxan oder Tetrahydrofuran, oder eines Gemisches davon verwendet. Dabei wendet man vorzugsweise geeignete Kondensations mittel, wie Alkalimetallcarbonate oder -hydrogencarbonate, z.B. Natrium- oder Kaliumcarbonat oder -hydrogencarbonat (üblicherweise zusammen mit einem Sulfat), oder organische Basen, wie üblicherweise sterisch gehinderte Triniederalkylamine, z.B. N,N-Diisopropyl-N-ethyl-amin (vorzugsweise zusammen mit Halogensulfonsäure-niederalkylestern oder gegebenenfalls halogensubstituierten Methansulfonsäureniederalkylestern) an, wobei unter Kühlen, bei Raumtemperatur oder unter Erwärmen, z.B. bei Temperaturen von etwa -20° C bis etwa 50° C, und, wenn notwendig, in einem geschlossenen Gefäss und/oder

in einer Inertgas-, z.B. Stickstoffatmosphäre, gearbeitet wird.

Weitere Mittel zur Veresterung sind entsprechende trisubstituierte Oxoniumsalze (sogenannte Meerweinsalze), oder disubstituierte Carbenium- oder Haloniumsalze, worin die Substituenten die verethernden Reste sind, beispielsweise Triniederalkyloxoniumsalze, sowie Diniederalkoxycarbenium- oder Diniederalkylhaloniumsalze, insbesondere die entsprechenden Salze mit komplexen, fluorhaltigen Säuren, wie die entsprechenden Tetrafluorborate, Hexafluorphosphate, Hexafluorantimonate, oder Hexachlorantimonate. Solche Reagentien sind z.B. Trimethyloxonium- oder Triethyloxoniumhexafluorantimonat, -hexachlorantimonat, -hexafluorphosphat oder -tetrafluorborat, Dimethoxycarbeniumhexafluorphosphat oder Dimethylbromoniumhexafluorantimonat. Man verwendet diese Mittel vorzugsweise in einem inerten Lösungsmittel, wie einem Ether oder einem halogenierten Kohlenwasserstoff, z.B. Diethylether, Tetrahydrofuran oder Methylenchlorid, oder in einem Gemisch davon, wenn notwendig, in Gegenwart einer Base, wie einer organischen Base, z.B. eines, vorzugsweise sterisch gehinderten, Triniederalkylamins, z.B. N,N-Diisopropyl-N-ethyl-amin, und unter Kühlen, bei Raumtemperatur oder unter leichtem Erwärmen, z.B. bei etwa -20°C bis etwa 50°C, wenn notwendig, in einem geschlossenen Gefäss und/oder in einer Inertgas-, z.B. Stickstoffatmosphäre.

Eine bevorzugte Ausführungsform dieser Veresterungsreaktion ist die Umsetzung eines Cäsiumsalzes einer Verbindung der Formel I, worin $R^2$ für Carboxy steht, mit dem als Veresterungskomponente gewünschten Alkohol, worin die Hydroxylgruppe in reaktionsfähiger veresterter Form vorliegt.

Salze von Verbindungen der Formel I können in an sich bekannter Weise durch Umsetzung mit einer geeigneten Base hergestellt werden, z.B. durch Behandeln mit geeigneten Metallverbindungen, wie Alkalimetallsalzen von geeigneten organischen Carbonsäuren, z.B. dem Natriumsalz der α-Ethyl-capronsäure, oder mit geeigneten anorganischen Alkali- oder Erdalkalimetallsalzen, insbesondere solchen, die sich von einer schwachen und vorzugsweise flüchtigen Säure ableiten, z.B. Natriumhydrogencarbonat, oder mit Ammoniak oder einem geeigneten organischen Amin bilden, wobei man vorzugsweise stöchiometrische Mengen oder nur einen kleinen Ueberschuss des salzbildenden Mittels verwendet.

Metall- und Ammoniumsalze können in üblicher Weise in die freien Verbindungen übergeführt werden, z.B. durch Behandeln mit geeigneten Säuren.

Gemische von Isomeren können in an sich bekannter Weise, z.B. durch fraktionierte Kristallisation, Chromatographie etc. in die einzelnen Isomeren aufgetrennt werden.

Die oben beschriebenen Verfahren, inklusive die Verfahren zur Abspaltung von Schutzgruppen und die zusätzlichen Verfahrensmassnahmen, werden, wenn nicht anders angegeben, in an sich bekannter Weise, z.B. in Anwesenheit von vorzugsweise inerten Lösungs- und Verdünnungsmitteln, wenn notwendig, in Anwesenheit von Kondensationsmitteln oder Katalysatoren, bei erniedrigter oder erhöhter Temperatur, z.B. in einem Temperaturbereich von etwa -20°C bis etwa +120°C, insbesondere von etwa 0°C bis etwa +70°C, vorzugsweise von etwa +10°C bis etwa +40°C, hauptsächlich bei Raumtemperatur, in einem geschlossenen Gefäss und/oder in einer Inertgas-, z.B. Stickstoffatmosphäre, durchgeführt.

Dabei sind unter Berücksichtigung aller im Molekül befindlichen Substituenten, wenn erforderlich, z.B. bei Anwesenheit leicht hydrolysierbarer Reste, besonders schonende Reaktionsbedingungen, wie kurze Reaktionszeiten, Verwendung von milden sauren oder basischen Mitteln in niedriger Konzentration, stöchiometrische Mengenverhältnisse, Wahl geeigneter Katalysatoren, Lösungsmittel, Temperatur- und/oder Druckbedingungen, anzuwenden.

Die Erfindung betrifft auch diejenigen Ausführungsformen des Verfahrens, bei denen man von einer auf irgendeiner Stufe des Verfahrens als Zwischenprodukt erhältlichen Verbindung ausgeht und die fehlenden Verfahrensschritte durchführt oder das Verfahren auf irgendeiner Stufe abbricht oder einen Ausgangsstoff in Form eines reaktionsfähigen Derivats oder Salzes verwendet. Dabei geht man vorzugsweise von solchen Ausgangsstoffen aus, die verfahrensgemäss zu den oben als besonders wertvoll beschriebenen Verbindungen führen.

Neue Ausgangsstoffe und/oder Zwischenprodukte sowie Verfahren zu ihrer Herstellung sind ebenfalls Gegenstand der vorliegenden Erfindung. Vorzugsweise werden solche Ausgangsstoffe verwendet und die Reaktionsbedingungen so gewählt, dass man zu den in dieser Anmeldung als besonders bevorzugt aufgeführten Verbindungen gelangt.

Die Erfindung betrifft auch pharmazeutische Präparate, die eine wirksame Menge, insbesondere eine zur Prophylaxe oder Therapie von Virusinfektionen oder eine antiallergisch oder antiinflammatorisch wirksame Menge, der Aktivsubstanz zusammen mit pharmazeutisch verwendbaren Trägerstoffen enthalten, die sich zur topischen, z.B. intranasalen, enteralen, z.B. oralen oder rektalen, oder parenteralen Verabreichung eignen, und anorganisch oder organisch, fest oder flüssig sein können. So verwendet man Tabletten oder Gelatine kapseln, welche den Wirkstoff zusammen mit Verdünnungsmitteln, z.B. Lactose, Dextrose, Sukrose, Mannitol, Sorbitol, Cellulose und/oder Glycerin, und/oder Schmiermitteln, z.B. Kieselerde, Talk, Stearinsäure oder Salze davon, wie Magnesium- oder Calciumstearat, und/oder Polyethylenglykol, enthalten. Tabletten können ebenfalls Bindemittel, z.B. Magnesiumaluminiumsilikat, Stärken, wie Mais-, Weizen-oder Reisstärke, Gelatine, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel enthalten. Ferner kann man die pharmakologisch wirksamen Verbindungen der vorliegenden Erfindung in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die

Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz-und/oder Emulgiermittel, Löslichkeitsvermittler, Salze zur Regulierung des osmotischen Drucks und/oder Puffer enthalten. Die vorliegenden pharmazeutischen Präparate, die, wenn erwünscht, weitere pharmakologisch wirksame Stoffe, wie Antibiotika, enthalten können, werden in an sich bekannter Weise, z.B. mittels konventioneller Misch-, Granulier-, Dragier-, Lösungs- oder Lyophilisierungsverfahren, hergestellt und enthalten von etwa 0,01 % bis 20 %, insbesondere von etwa 0,1 % bis etwa 10 %, in erster Linie zwischen 0,5 % und 5 %, des bzw. der Aktivstoffe, wobei eine Wirkstoffkonzentration unterhalb von 1 % insbesondere für topisch zu applizierende Präparate geeignet ist.

Als topisch zu applizierende Darreichungsformen sind die folgenden bevorzugt: Crèmes, Salben oder Pasten, z.B. Salben zur intranasalen Applikation oder Lippenstifte, oder vorzugsweise isotonische, sterile und physiologisch verträgliche Lösungen, z.B. Augentropfen, vorzugsweise in Mikrobehältern zur einmaligen Verwendung, oder Sprays zur Anwendung im Mund- und Rachenraum.

Besonders geeignet sind die in den Beispielen beschriebenen pharmazeutischen Präparate.

Die nachfolgenden Beispiele illustrieren die Erfindung, ohne sie in irgendeiner Form einzuschränken. Die $R_f$-Werte werden, wenn nicht anders angegeben, auf Kieselgeldünnschichtplatten (Firma Merck, Darmstadt, Deutschland) in folgenden Lösungsmittelsystemen ermittelt:

A: Chloroform-Methanol-Wasser (70:30:5)

B: Chloroform-Methanol (7:3).

Im folgenden bedeutet z.B. "$R_f$(A)", dass der $R_f$-Wert im System A ermittelt wurde. Die Flecke, die phospholipidhaltige Produkte enthalten, können im Dünnschichtchromatogramm mit modifiziertem Zinzadze-Reagenz [spezifisch für Phospholipide; V.E. Vaskovsky und E.Y. Kostetsky, Journal of Lipid Reserach 9, 396 (1968)] sichtbar gemacht werden. Das Verhältnis der Laufmittel in den verwendeten Laufmittelgemischen zueinander ist in Volumenanteilen (V/V), Temperaturen sind in Grad Celsius angegeben. Die Konzentration, c, der Substanz im Lösungsmittel(gemisch) ist im Falle der optischen Drehung in Prozent (Gewicht/Volumen) angegeben. Bei in analoger Weise durchgeführten Beispielen stehen die Mengen nicht aufgeführter Reaktanden, Reagenzien und Lösungsmittel im gleichen Verhältnis zu den entsprechenden Mengen im Beispiel, auf welches Bezug genommen wird, wie die angegebenen Molmengen einander entsprechender Edukte im Analogbeispiel und in dem in Bezug genommenen Beispiel zueinander.

Abkürzungen:

Smp. = Schmelzpunkt

Zers. = Zersetzung

Beispiel 1: Eine Lösung von 1,40 g (2,6 mMol) 4-(2,3,4,6-Tetra-O-acetyl-β-D-glucopyranosyloxy)-buttersäure in einem Gemisch von 70 ml Chloroform und 35 ml Isopropanol wird bei 22° nacheinander mit 1,8 g (2,6 mMol) 2-(1,2-Dipalmitoyl-sn-glycero-3-hyroxyphosphoryloxy)-ethylamin, 518 mg (3,38 mMol) 1-Hydroxybenzotriazol, 0,38 ml (2,73 mMol) Triethylamin, 700 mg (3,4 mMol) N,N'-Dicyclohexylcarbodiimid und 1,2 ml Wasser versetzt. Man rührt 20 Std. bei 22° und dampft dann im Vakuum ein. Der ölige Rückstand wird in 200 ml Chloroform aufgenommen und die Lösung zunächst mit 200 ml 0,3molarer Natriumphospat-Pufferlösung, pH 7,0, und dann mit gesättigter Natriumchloridlösung gewaschen. Nach dem Extrahieren der wässrigen Phasen mit weiteren 50 ml Chloroform werden die organischen Phasen vereinigt, über Natriumsulfat getrocknet, mit 6 ml 3molarer methanolischer Natrium-α-ethylhexanoat-Lösung versetzt und im Vakuum eingedampft. Aus dem in 50 ml Aceton aufgenommenen Eindampfrückstand gewinnt man bei 0° farblose Kristalle, die durch Chromatographie an 200 g Kieselgel mit Chloroform-Methanol-Wasser (60:40:3) gereinigt werden. Man erhält 4-(2,3,4,6-Tetra-O-acetyl-β-D-glucopyranosyloxy)-buttersäure-2-(1,2-dipalmitoyl-sn-glycero-3-hydroxyphosphoryloxy)-ethylamid-natriumsalz; $R_f$(B) = 0,60, $[\alpha]_D^{20}$ = -3,0° (Chloroform:Methanol = 1:1; c = 1).

Das Ausgangsmaterial erhält man folgendermassen:

Stufe 1.1: 49,5 g (120 mMol) 2,3,4,6-Tetra-O-acetyl-α-D-glucopyranosylbromid und 30,2 g (143 mMol) Silbersalz der 4-Hydroxy-buttersäure [G. Wulff et al., Chemische Berichte 104, 1387-1399 (1971)] werden in 500 ml absolutem Toluol aufgenommen und unter Lichtausschluss 40 Min. bei 22° gerührt. Man filtriert das entstandene Silberbromid ab und dampft das farblose Filtrat zum öligen Rückstand ein. Dieser wird in 750 ml Chloroform gelöst und 2mal mit je 750 ml 0,5molarer Natriumhydrogencarbonatlösung extrahiert. Die wässrige Phase wird mit 500 ml Chloroform gewaschen und von pH 8,3 auf pH 3 mit Phosphorsäure angesäuert. Man extrahiert 2mal mit je 500 ml Chloroform, trocknet die Extrakte über Natriumsulfat und dampft ein. Durch Kristallisation aus Diethylether erhält man 4-(2,3,4,6-Tetra-O-acetyl-β-D-glucopyranosyloxy-buttersäure; Smp. 98-100°.

Beispiel 2: Analog Beispiel 1 erhält man aus 693 mg (2,60 mMol) 4-(β-D-Glucopyranosyloxy)-buttersäure 4-(β-D-Glucopyranosyloxy)-buttersäure-2-(1,2-dipalmitoyl-sn-glycero-3-hydroxyphosphoryloxy)-ethylamid-natriumsalz; $R_f$(A) = 0,27, $[\alpha]_D^{20}$ = -1,8° (Chloroform:Methanol = 1:1; c = 1).

Das Ausgangsmaterial erhält man folgendermassen:

Stufe 2.1: 1,5 g (3,4 mMol) 4-(2,3,4,6-Tetra-O-acetyl-β-D-glucopyranosyloxy)-buttersäure (s. Stufe 1.1) werden in 13 ml Chloroform aufgenommen und bei -10° mit 22 ml Methanol, enthaltend 17,3 mMol Natriummethylat (durch Auflösen von 400 mg Natrium erhalten), versetzt. Man rührt 1 1/2 Stunden bei 0° und 1 Stunde bei 22°. Das Gemisch wird auf 0° gekühlt, mit 2 ml Wasser versetzt und mit 1molarer Salzsäure auf pH 3,0 gestellt. Man dampft im Vakuum ein, nimmt den Rückstand in warmem Ethanol auf und filtriert das entstandene

Natriumchlorid ab. Nach Eindampfen des Filtrats erhält man 4-(β-D-Glucopyranosyloxy)-buttersäure.

Beispiel 3: Analog Beispiel 1 erhält man aus 1000 mg (2,30 mMol) 4-(2,3,4,6-Tetra-O-acetyl-α-D-mannopyranosyloxy)-buttersäure 4-(2,3,4,6-Tetra-O-acetyl-α-D-mannopyranosyloxy)-buttersäure-2-(1,2-dipalmitoyl-sn-glycero-3-hydroxyphosphoryloxy)-ethylamidnatriumsalz; $R_f(B) = 0{,}40$, $[\alpha]_D^{20} = +3{,}2°$ (Chloroform: Methanol = 1:1; c = 1).

Das Ausgangsmaterial erhält man folgendermassen:

Sufe 3.1: Analog Stufe 5.1 erhält man aus 10,0 g Penta-O-acetyl-D-mannose 2,3,4,6-Tetra-O-acetyl-D-mannosylbromid.

Stufe 3.2: Aus 57,1 g (139 mMol) 2,3,4,6-Tetra-O-acetyl-D-mannosylbromid erhält man analog Stufe 1.1 4-(2,3,4,6-Tetra-O-acetyl-α-D-mannopyranosyloxy)-buttersäure; Smp. 121=123°.

Beispiel 4: Analog Beispiel 1 erhält man aus 580 mg (2,18 mMol) 4-(β-D-Galactopyranosyloxy)-buttersäure 4-(β-D-Galactopyranosyloxy)-buttersäure-2-(1,2-dipalmitoyl-sn-glycero-3-hydroxyphosphoryloxy)-ethylamidnatriumsalz; $R_f(A) = 0{,}25$, $[\alpha]_D^{20} = +2{,}2°$ (Chloroform:Methanol = 1:1; c = 1).

Das Ausgangsmaterial erhält man folgendermassen:

Stufe 4.1: Aus 49,3 g (120 mMol) 2,3,4,6-Tetra-O-acetyl-α-D-galactopyranosylbromid erhält man analog Stufe 1.1 4-(2,3,4,6-Tetra-O-acetyl-β-D-galactopyranosyloxy)-buttersäure; Smp. 57-60°.

Stufe 4.2: Aus 4-(2,3,4,6-Tetra-O-acetyl-β-D-galactopyranosyloxy)-buttersäure erhält man analog Stufe 2.1 4-(β-D-Galactopyranosyloxy)-buttersäure.

Beispiel 5: Analog Beispiel 1 erhält man aus 700 mg (1,63 mMol) 4-(β-D-Cellobiopyranosyloxy)-buttersäure 4-(β-D-Cellobiopyranosyloxy)-buttersäure-2-(1,2-dipalmitoyl-sn-glycero-3-hydroxyphosphoryloxy)-ethylamidnatriumsalz; $R_f(A) = 0{,}20$, $[\alpha]_D^{20} = -1{,}1°$, (Chloroform:Methanol = 1:1; c = 1).

Das Ausgangsmaterial erhält man folgendermassen:

Stufe 5.1: Analog der Vorschrift von E. Fischer, Ber. Dtsch. Chem. Ges. 49, 584-585 (1916) werden 27,8 g (41 mMol) Octa-O-acetyl-D-cellobiose in 60 ml Eisessig aufgenommen und mit 40 ml Methylenchlorid und 41 ml einer 33%igen Bromwasserstofflösung in Eisessig versetzt. Nach 15 Minuten Rühren bei 22° geht die Suspension in Lösung. Nach insgesamt 2 1/2 Stunden Rühren bei 22° wird die Lösung auf 500 ml Eiswasser geleert. Man extrahiert 3mal mit je 500 ml Chloroform. Die organischen Phasen werden mehrmals mit Eiswasser neutralgewaschen, dann über Magnesiumsulfat getrocknet und eingedampft. Durch Kristallisation aus Diethylether erhält man Hepta-O-acetyl-α-D-cellobiopyranosylbromid; Smp. 190-191°.

Stufe 5.2: Analog Stufe 1.1 erhält man aus 16,0 g (22,9 mMol) Hepta-O-acetyl-α-D-cellobiopyranosylbromid 4-(Hepta-O-acetyl-β-D-cellobiopyranosyloxy)-buttersäure.

Stufe 5.3: Analog Stufe 2.1 erhält man aus 4-(Hepta-O-acetyl-β-D-cellobiopyranosyloxy)-buttersäure 4-(β-D-Cellobiopyranosyloxy)-buttersäure.

Beispiel 6: Analog Beispiel 1 erhält man aus 700 mg (1,63 mMol) 4-(β-D-Lactopyranosyloxy)-buttersäure 4-(β-D-Lactopyranosyloxy)-buttersäure-2-(1,2-dipalmitoyl-sn-glycero-3-hydroxyphosphoryloxy)-ethylamidnatriumsalz; $R_f(A) = 0{,}23$, $[\alpha]_D^{20} = +2{,}1°$, (Chloroform:Methanol = 1:1; c = 1).

Das Ausgangsmaterial erhält man folgendermassen:

Stufe 6.1: Aus 6,9 g Octa-O-acetyl-β-D-lactopyranose erhält man analog Stufe 5.1 Hepta-O-acetyl-α-D-lactopyranosylbromid; Smp. 139-140°.

Stufe 6.2: Aus 16,0 g (22,9 mMol) Hepta-O-acetylα-D-laotopyranosylbromid erhält man analog Stufe 1.1 4-(Hepta-O-acetyl-β-D-lactopyranosyloxy)-buttersäure.

Stufe 6.3: 1,5 g rohe 4-(Hepta-O-acetyl-β-D-lactopyranosyloxy)-buttersäure werden in 15 ml Chloroform gelöst und nach Versetzen mit 0,83 ml einer 3molaren methanolischen Natrium-α-ethylhexanoat-Lösung 10 Min. gerührt. Man dampft ein, digeriert mehrmals mit Diethylether und erhält kristallines 4-(Hepta-O-acetyl-β-D-lactopyranosyloxy)-buttersäure-natriumsalz.

Stufe 6.4: Aus 4-(Hepta-O-acetyl-β-D-lactopyranosyloxy)-buttersäure-natriumsalz erhält man analog Stufe 2.1 4-(β-D-Lactopyranosyloxy)-buttersäure.

Beispiel 7: Analog Beispiel 1 erhält man aus 700 mg (1,63 mMol) 4-(β-D-Maltopyranosyloxy)-buttersäure 4-(β-D-Maltopyranosyloxy)-buttersäure-2-(1,2-dipalmitoyl-sn-glycero-3-hydroxyphosphoryloxy)-ethylamidnatriumsalz; $R_f(A) = 0{,}33$, $[\alpha]_D^{20} = +28{,}4°$ (Chloroform:Methanol = 1:1; c = 1).

Das Ausgangsmaterial erhält man folgendermassen:

Stufe 7.1: Aus 37,0 g Octa-O-acetyl-D-maltopyranose erhält man analog Stufe 5.1 Hepta-O-acetyl-α-D-maltopyranosylbromid.

Stufe 7.2: Aus Hepta-O-acetyl-α-D-maltopyranosylbromid erhält man analog Stufe 1.1 4-(Hepta-O-acetyl-β-D-maltopyranosyloxy)-buttersäure.

Stufe 7.3: Aus 4-(Hepta-O-acetyl-β-D-maltopyranosyloxy)-buttersäure erhält man analog Stufe 6.3 4-(Hepta-O-acetyl-β-D-maltopyranosyloxy)-buttersäure-natriumsalz.

Stufe 7.4: Aus 4-(Hepta-O-acetyl-β-D-maltopyranosyloxy)-buttersäure-natriumsalz erhält man analog Stufe 2.1 4-(β-D-Maltopyranosyloxy)-buttersäure.

Beispiel 8: Analog Beispiel 1 erhält man aus 600 mg (2,40 mMol) 4-(β-L-Fucopyranosyloxy)-buttersäure 4-(β-L-Fucopyranosyloxy)-buttersäure-2-(1,2-dipalmitoyl-sn-glycero-3-hydroxyphosphoryloxy)-ethylamid-natriumsalz; $R_f(A) = 0{,}37$, $[\alpha]_D^{20} = +9{,}4°$ (Chloroform:Methanol = 1:1; c = 1).

Das Ausgangsmaterial erhält man folgendermassen:

Stufe 8.1: Aus 27,6 g Tetra-O-acetyl-L-fucose erhält man analog Stufe 5.1 Tri-O-acetyl-L-fucosylbromid.

Stufe 8.2: Aus 26,5 g (75,0 mMol) Tri-O-acetyl-L-fucosylbromid erhält man analog Stufe 1.1 4-(2,3,4-Tri-O-a-

cetyl-β-L-fucopyranosyloxy)-buttersäure.

Stufe 8.3: Aus 4-(2,3,5-Tri-O-acetyl-β-L-fucopyranosyloxy)-buttersäure erhält man analog Stufe 6.3 4-(2,3,4-Tri-O-acetyl-β-L-fucopyranosyloxy)-buttersäure-natriumsalz.

Stufe 8.4: Aus 4-(2,3,4-Tri-O-acetyl-β-L-fucopyranosyloxy)-buttersäure-natriumsalz erhält man analog Stufe 2.1 4-(β-L-Fucopyranosyloxy)-buttersäure.

Beispiel 9: Analog Beispiel 1 erhält man aus 4-(2-Acetylamino-2-desoxy-β-D-glucopyranosyloxy)-buttersäure 4-(2-Acetylamino-2-desoxy-β-D-glucopyranosyloxy)-buttersäure-2-(1,2-dipalmitoyl-sn-glycero-3-hydroxyphosphoryloxy)-ethylamid-natriumsalz.

Das Ausgangsmaterial erhält man folgendermassen:

Stufe 9.1: Aus 2-Acetylamino-3,4,6-tri-O-acetyl-2-desoxy-α-D-glucopyranosylbromid [hergestellt nach J.W. Gillard et al., Tetrahedron Lett. 22, 513 (1981)] erhält man analog Stufe 1.1 4-(2-Acetylamino-3,4,6-tri-O-acetyl-2-desoxy)-β-D-glucopyranoxyloxy)-buttersäure.

Stufe 9.2: Aus 4-(2-Acetylamino-3,4,6-tri-O-acetyl-2-desoxy)-β-D-glucopyranosyloxy)-buttersäure erhält man analog Stufe 2.1 4-(2-Acetylamino-2-desoxy-β-D-glucopyranosyloxy)-buttersäure.

Beispiel 10: Analog Beispiel 1 erhält man aus 518 mg (1,60 mMol) N-[4-(β-D-Galactopyranosyloxy)-butyryl]-glycin N-[4-(β-D-Galactopyranosyloxy)-butyryl]-glycin-2-(1,2-dipalmitoyl-sn-glycero-3-hydroxyphosphoryloxy)-ethylamid-natriumsalz; $R_f(A) = 0,27$, $[\alpha]_D^{20} = +1,4°$ (Chloroform:Methanol = 1:1; c = 1).

Das Ausgangsmaterial erhält man folgendermassen:

Stufe 10.1: Eine Lösung von 640 mg (2,59 mMol) 4-(β-D-Galactopyranosyloxy)-buttersäure in 4 ml N,N-Dimethylformamid und 4 ml Chloroform wird mit 516 mg (3,37 mMol) 1-Hydroxy-benztriazol und 695 mg (3,37 mMol) N,N'-Dicyclohexylcarbodiimid versetzt und 2 Stunden bei 22° gerührt. Dazu gibt man eine aus 194 mg (2,59 mMol) Glycin und 2 ml Wasser durch Zugabe von normaler Natronlauge bei pH 10,6 bereitete Lösung und rührt anschliessend 18 Stunden bei 22°. Das Gemisch wird im Hochvakuum eingedampft, der Rückstand in wenig N,N-Dimethylformamid aufgenommen und nach kurzem Rühren bei 0° durch Filtration vom Unlöslichen befreit. Das eingedampfte Filtrat gibt beim Digerieren mit Aceton 618 mg N-[4-(β-D-Galactopyranosyloxy)-butyryl]-glycin-natriumsalz als weisses Pulver.

Stufe 10.2: Eine Lösung von 600 mg N-[4-(β-D-Galactopyranosyloxy)-butyryl]-glycin-natriumsalz in 10 ml Methanol-Wasser (1:1) wird bei 0° mit 1normaler Salzsäure auf pH 2,5 gestellt und im Vakuum eingedampft. Der in warmem Ethanol gelöste Rückstand wird klarfiltriert und im eingedampften Filtrat das N-[4-(β-D-Galactopyranosyloxy)-butyryl]-glycin erhalten.

Beispiel 11: Analog Beispiel 1, jedoch unter Verwendung von Chloroform-Methanol-Wasser (70:30:5) als Eluiermittel bei der Chromatographie, erhält man aus 591 mg (1,38 mMol) 4-(β-D-Cellobiopyranosyloxy)-buttersäure (siehe Stufe 5.3) und 800 mg (1,38 mMol) 2-(1,3-Di-n-dodecanoyl-glycero-2-hydroxyphosphoryloxy)-ethylamin 4-(β-D-Cellobiopyranosyloxy)-buttersäure-2-(1,3-di-n-dodecanoyl-glycero-2-hydroxyphosphoryloxy)-ethylamid-natriumsalz; $R_f(A) = 0,25$, $[\alpha]_D^{20} = -6,9°$ (Chloroform:Methanol = 1:1; c = 1).

Beispiel 12: Eine Lösung von 1000 mg (3,26 mMol) 4-(α-D-Mannopyranosylthio)-buttersäure, 0,74 g (4,9 mMol) 1-Hydroxy-benzotriazol (enthaltend 12 % Wasser) und 1,4 g (6,6 mMol) N,N'-Dicyclohexylcarbodiimid in 50 ml N,N-Dimethyl-formamid wird 3 Stunden bei 22° gerührt. Man dampft das Gemisch im Hochvakuum ein und digeriert den öligen Rückstand 3mal mit je 50 ml Diethylether. Der erhaltene farblose, kristalline Rückstand wird in 30 ml Isopropanol aufgenommen und dazu bei 22° innerhalb von 10 Min. eine Lösung von 1,86 g (2,61 mMol) 2-(1,2-Dipalmitoyl-sn-glycero-3-hydroxyphosphoryloxy)-ethylamin-natriumsalz in 50 ml Chloroform-Isopropanol (9:1) getropft. Man rührt anschliessend 30 Minuten, gibt 8 ml Wasser zu und rührt noch 2 Stunden bei 22°. Der Ansatz wird analog Beispiel 1 mit 6 ml 3molarer Natrium-α-ethyl-hexanoat-Lösung versetzt, eingedampft, aus Aceton kristallisiert und dann wie dort chromatographiert. Man erhält 4-(α-D-Mannopyranosylthio)-buttersäure-2-(1,2-dipalmitoyl-sn-glycero-3-hydroxyphosphoryloxy)-ethylamid-natriumsalz; $R_f(A) = 0,41$, $[\alpha]_D^{20} = +67°$ (Methanol; c = 0,66).

Das Ausgangsmaterial erhält man wie folgt:

Stufe 12.1: Ein Gemisch von 90 g (0,185 Mol) S-(2,3,4,6-Tetra-O-acetyl-α-D-mannopyranosyl)-isothiuroniumbromid [K.L. Matta et al., Carbohydrate Research 43, 101 (1975); P.L. Durette et al., Carbohydrate Research 81, 261 (1980)], 42,6 g (0,199 Mol) 4-Iodbuttersäure, 29,6 g (0,215 Mol) Kaliumcarbonat und 37 g (0,187 Mol) Kaliumdisulfit ($2\ K_2S_2O_5 \cdot 3\ H_2O$) in 150 ml Aceton und 150 ml Wasser wird 1 Stunde bei 22° gerührt. Man gibt 700 ml 5%ige Salzsäure und 700 ml Chloroform zu und rührt 5 Minuten. Die organische Phase wird abgetrennt, über Natriumsulfat getrocknet und eingedampft. Der ölige Rückstand enthält 4-(2,3,4,6-Tetra-O-acetyl-α-D-mannopyranosylthio)-buttersäure.

Stufe 12.2: Aus 4-(2,3,4,6-Tetra-O-acetyl-α-D-mannopyranosylthio)-buttersäure erhält man analog Stufe 2.1 4-(α-D-Mannopyranosylthio)-buttersäure.

Beispiel 13: Analog Beispiel 12 erhält man aus 4-(α-D-Mannopyranosyloxy)-buttersäure (siehe Stufe 3.2) 4-(α-D-Mannopyranosyloxy)-buttersäure-2-(1,2-dipalmitoyl-sn-glycero-3-hydroxyphosphoryloxy)-ethylamid-natriumsalz.

Beispiel 14: Analog Beispiel 2 erhält man aus 4-(β-D-Glucopyranosyloxy)-buttersäure und entsprechenden Phosphatidylverbindungen 4-(β-D-Glucopyranosyloxy)-butyryl]-O-(1,2-dioleoyl-sn-glycero-3-hydroxyphosphoryl)-L-serin-dinatriumsalz, N-[4-(β-D-Glucopyranosyloxy)-butyryl]-O-(1,3-di-n-dodecanoyl-glycero-2-hydroxyphosphoryl)-L-serin-dinatriumsalz, 4-(β-D-Glucopyranosyloxy)-buttersäure-2-(2-n-decanoyl oxyethyloxy-hydroxyphosphoryloxy)-ethylamid-natriumsalz, 4-(β-D-Glucopyranosyloxy)-buttersäure-2-(1,2-distearoyl-sn- glycero-3-hydroxyphosphoryloxy)-ethylamid-natriumsalz, 4-(β-D-Glucopyranosy-

loxy)-buttersäure-2-(1,2-diarachinoyl-sn-glycero-3-hydroxyphosphoryloxy)-ethylamid-natriumsalz, 4-(β-D-Glucopyranosyloxy)-buttersäure-2-(1,2-dimyristoyl-sn-glycero-3-hydroxyphosphoryloxy)-ethylamid-natriumsalz, 4-(β-D-Glucopyranosyloxy)-buttersäure-2-(1,2-dilauroyl-sn-glycero-3-hydroxyphosphoryloxy)-ethylamid-natriumsalz, 4-(β-D-Glucopyranosyloxy)-buttersäure-2-(1-palmitoyl-2-oleoyl-sn-glycero-3-hydroxyphosphoryloxy)-ethylamid-natriumsalz und 4-(β-D-Glucopyranosyloxy)-buttersäure-2-(1-palmitoyl-glycero-3-hydroxyphosphoryloxy)-ethylamid-natriumsalz.

Beispiel 15: Nasensalbe

Zusammensetzung

4-(β-D-Maltopyranosyloxy)-buttersäure-2-(1,2-dipalmitoyl-sn-glycero-3-hydroxyphosphoryloxy)-ethylamid-natriumsalz 0,50 g
Paraffinöl dickflüssig 20,00 g
weisses Vaselin 30,00 g
Wollfett, wasserfrei 40,00 g
entmineralisiertes Wasser 19,50 g

Herstellung

Die Fettphase, bestehend aus Paraffinöl, Vaselin und Wollfett, wird zusammengeschmolzen. Die wässrige Lösung des Wirkstoffs wird bei ca. 50°C in die Fettphase eingearbeitet.

Beispiel 16: Analog Beispiel 1 erhält man aus 1,344 g (4,57 mMol) 2-(2-Acetylamino-2-desoxy-β-D-glucopyranosyloxy)-essigsäure 2-(2-Acetylamino-2-desoxy-β-D-glucopyranosyloxy)-essigsäure-2-(1,2-dipalmitoyl-sn-glycero-3-hydroxyphosphoryloxy)-ethylamid-natriumsalz; $R_f(A) = 0,37$, $[\alpha]_D^{20} = -15,2°$ (Chloroform:Methanol = 1:1; c = 1).

Das Ausgangsmaterial erhält man folgendermassen:

Stufe 16.1: Eine Lösung von 300 mg (1,85 mMol) Eisen(III)-chlorid (wasserfrei) in 6 ml absolutem Methylenchlorid wird mit einer Lösung von 600 mg (1,82 mMol) 2-Methyl-(3,4,6-tri-O-acetyl-1,2-didesoxy-α-D-glucopyrano)-[2,1-d]-2-oxazolin der Formel

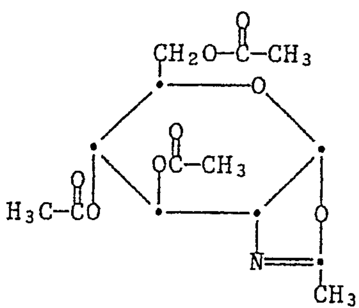

[hergestellt nach V.K. Srivastava, Carbohydrate Research 103, 286 (1982)] und 605 mg (3,64 mMol) Glykolsäurebenzylester in 6 ml Methylenchlorid versetzt und 21 Stunden bei 22° gerührt. Man gibt 8 ml kalte, gesättigte Natriumbicarbonatlösung zu, verdünnt mit 100 ml Chloroform und filtriert klar. Die organische Phase des Filtrats gibt nach Trocknen über Magnesiumsulfat und Eindampfen ein gelbes Oel, das durch Digerieren einer Ethylacetat-Lösung mit Hexan und durch Chromatographie an Kieselgel gereinigt wird. Man erhält 2-(2-Acetylamino-3,4,6-tri-O-acetyl-2-desoxy-β-D-glucopyranosyloxy)-essigsäure-benzylester als farbloses Pulver.

Stufe 16.2: Eine Lösung 2,54 g (6,87 mMol) 2-(2-Acetylamino-3,4,6-tri-O-acetyl-2-desoxy-β-D-glucopyranosyloxy)-essigsäurebenzylester in 60 ml absolutem Methanol wird bei 0° mit einer durch Auflösen von 472 mg (27,5 mMol) Natrium in 20 ml absolutem Methanol hergestellten Natriummethylat-Lösung versetzt und 2 1/2 Stunden bei 0° sowie 1 Stunde bei 22° gerührt. Das Gemisch wird im Vakuum eingedampft, der Rückstand in wenig Methanol gelöst und mit Diethylether gefällt. Das erhaltene rohe Natriumsalz wird in 60 ml Methanol-Wasser (2:1) gelöst und bei 0° mit einnormaler Salzsäure auf pH 3,1 angesäuert. Man dampft ein, nimmt in Ethanol-Chloroform (1:1) auf, filtriert klar, dampft erneut ein, nimmt in Ethanol auf, klärt und fällt mit Ethylacetat-Hexan aus. Man erhält 2-(2-Acetylamino-2-desoxy-β-D-glucopyranosyloxy)-essigsäure als weissliches Pulver.

Beispiel 17: Weibliche Balb/c-Mäuse (im Falle von Wirksubstanz A) bzw. MF-2f SPF-Mäuse (im Falle von Wirksubstanz B) mit einem Körpergewicht von 14-16 g werden unter leichter Narkose mit einem Gemisch aus gleichen Teilen Diethylether, Ethanol und Chloroform mit letalen Dosen (ungefähr eine $LD_{95}$) in Form von je 0,05 ml einer Suspension von Influenza A/Texas/1/77 Viren (Maus-adaptierter Stamm) intranasal infiziert.

Jeder Maus aus Gruppen von jeweils 10 dieser Mäuse wird zum unten angegebenen Zeitpunkt [Tage] bezogen auf den Tag der Infektion einmal (Einzeldosis) die in Tabelle 1 genannte Menge der jeweiligen Wirksubstanz in 0,05 bzw. in 0,2 ml einer 0,005 Gew.%igen Lösung von Carboxymethylcellulose-natriumsalz in

doppelt destilliertem, pyrogenfreiem Wasser im Falle von intranasaler bzw. oraler Applikation auf die in Tabelle 1 genannte Art appliziert.

Jeweils 20 der obengenannten infizierten Mäuse dienen zur Kontrolle, d.h. sie erhalten ein Placebo (0,005 Gew.%ige Lösung von Carboxymethylcellulose-natriumsalz).

Die intranasale Applikation der Wirksubstanz wird unter leichter Narkose mit einem Gemisch aus gleichen Teilen Diethylether, Ethanol und Chloroform durchgeführt.

Wirksubstanz A = 4-(β-D-Maltopyranosyloxy)-buttersäure-2-(1,2-dipalmitoyl-sn-glycero-3-hydroxyphosphoryloxy)-ethylamid-natriumsalz

Wirksubstanz B = 4-(α-D-Mannopyranosylthio)-buttersäure-2-(1,2-dipalmitoyl-sn-glycero-3-hydroxyphosphoryloxy)-ethylamid-natriumsalz

Die Resultate sind aus der nachfolgenden Tabelle ersichtlich.

| Wirksubstanz | Applikationsart | Applikationszeitpunkt [Tage] | Prozentsatz der 23 Tage (Wirksubstanz B) bzw. 24 Tage (Wirksubstanz A) nach der Infektion noch lebenden Mäuse in Abhängigkeit von der Wirksubstanzmenge [mg/kg], statistische Signifikanz * P ≤ 0,05, ** P ≤ 0,01 (Vierfelder-Test), n.t. = nicht geprüft | | | |
|---|---|---|---|---|---|---|
| | | | 0,1 | 0,01 | 0,001 | 0 = Kontrolle |
| A | oral | +7 | 80** | 70** | 50* | 5 |
| B | intra-nasal | -7 | 80** | 90** | 50* | 5 |

## Patentansprüche

1. Saccharidderivate der Formel I,

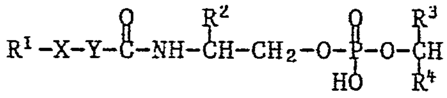

(I)

worin $R_1$ a) Aldohexosyl, b) D-Aldohexosyl, welches in 4- oder 6-Stellung glykosidisch mit D-Aldohexosyl verknüpft ist, c) Aldopentosyl, d) 6-Desoxy-aldohexosyl oder e) 2-Acetylamino-2-desoxy-D-aldohexosyl, wobei in den oben unter a) bis e) genannten Resten vorhandene freie Hydroxygruppen peracetyliert sein können, bedeutet, X Sauerstoff oder Schwefel bedeutet, Y Alkylen mit bis zu 10 C-Atomen, worin 1-3 nicht-endständige Methylengruppen durch Sauerstoff, Carbonylimino oder Carbonyloxy ersetzt sein können, bedeutet, $R^2$ Wasserstoff, Carboxy, Niederalkoxycarbonyl, Benzyloxycarbonyl oder Carbamoyl bedeutet, $R^3$ Wasserstoff und $R^4$ eine 1,2-Dihydroxy-ethyl-, 2-Hydroxy-ethyl- oder Hydroxymethyl-Gruppe, in der mindestens eine Hydroxygruppe mit einer unsubstituierten aliphatischen $C_{10-24}$-Carbonsäure verestert ist, und in der die andere Hydroxygruppe, falls vorhanden, frei oder mit einer aliphatischen $C_{2-24}$-Carbonsäure verestert ist, oder $R^3$ und $R^4$ je eine mit einer unsubstituierten aliphatischen $C_{10-24}$-Carbonsäure veresterte Hydroxymethylgruppe bedeuten, und Salze dieser Verbindungen.

2. Verbindungen der Formel I nach Anspruch 1, worin $R^3$ Wasserstoff und $R^4$ 1,2-Dihydroxy-ethyl, worin entweder nur die 2-Hydroxy-Gruppe oder beide Hydroxygruppen unabhängig voneinander mit einer $C_{10-24}$-Alkansäure oder einer $C_{18}$-Alkensäure verestert sind, bedeuten oder $R^3$ und $R^4$ je mit einer $C_{10-24}$-Alkansäure oder einer $C_{18}$-Alken säure verestertes Hydroxymethyl bedeuten und die übrigen Substituenten die in Anspruch 1 genannten Bedeutungen haben, und ihre Salze.

3. Verbindungen der Formel I nach Anspruch 1, worin $R^3$ Wasserstoff und $R^4$ 1,2-Dihydroxyethyl, worin beide Hydroxygruppen unabhängig voneinander mit einer $C_{10-24}$-Alkansäure oder einer $C_{18}$-Alkensäure verestert sind, bedeuten oder $R^3$ und $R^4$ je mit einer $C_{10-24}$-Alkansäure oder einer $C_{18}$-Alkensäure verestertes Hydroxymethyl bedeuten und die übrigen Substituenten die in Anspruch 1 genannten Bedeutungen haben, und ihre Salze.

4. Verbindungen der Formel I nach Anspruch 1, worin $R^3$ Wasserstoff und $R^4$ 1,2-Dihydroxyethyl, worin beide Hydroxygruppen unabhängig voneinander mit einer unverzweigten $C_{10-24}$-Alkansäure mit gerader Anzahl von C-Atomen oder einer unverzweigten $C_{18}$-Alkensäure mit 1-3 isolierten Doppelbindungen verestert sind, bedeuten oder $R^3$ und $R^4$ je mit einer unverzweigten $C_{10-24}$-Alkansäure mit gerader Anzahl von C-Atomen oder einer unverzweigten $C_{18}$-Alkensäure mit 1-3 isolierten Doppelbindungen verestertes Hydroxymethyl bedeuten und die übrigen Substituenten die in Anspruch 1 genannten Bedeutungen haben, und ihre Salze.

5. Verbindungen der Formel I nach einem der Ansprüche 1-4, worin Y unverzweigtes $C_1$-$C_5$-Alkylen oder den Rest $-CH_2-CH_2-CH_2-CO-NH-CH_2-$bedeutet, und ihre Salze.

6. Verbindungen der Formel I nach Anspruch 1, worin $R^1$ a) D-Aldohexosyl, welches in 4-Stellung glykosidisch mit D-Aldohexosyl verknüpft sein kann, b) 6-Desoxy-aldohexosyl oder c) 2-Acetylamino-2-desoxy-D-aldohexosyl, wobei in den oben unter a) bis c) genannten Resten vorhandene freie Hydroxygruppen peracetyliert sein können, bedeutet, X Sauerstoff oder Schwefel bedeutet, Y $C_{3-5}$-Alkylen, worin eine nicht endständige Methylengruppe durch Carbonylimino ersetzt sein kann, bedeutet, $R^2$ Wasserstoff oder Carboxy bedeutet, $R^3$ Wasserstoff und $R^4$ 1,2-Dihydroxy-ethyl, worin entweder nur die 2-Hydroxy-Gruppe oder beide Hydroxygruppen unabhängig voneinander mit einer unverzweigten $C_{10-24}$-Alkansäure oder einer unverzweigten $C_{18}$-Alkensäure verestert sind, bedeuten oder $R^3$ und $R^4$ je mit einer unverzweigten $C_{10-24}$-Alkansäure oder einer unverzweigten $C_{18}$-Alkensäure mit 1-3 isolierten Doppelbindungen verestertes Hydroxymethyl bedeuten, und ihre Salze.

7. Verbindungen der Formel I nach Anspruch 1, worin $R^1$ a) D-Aldohexosyl, worin freie Hydroxygruppen peracetyliert sein können, b) D-Aldohexosyl, welches in 4-Stellung glykosidisch mit D-Aldohexosyl verknüpft ist, c) 6-Desoxy-aldohexosyl oder d) 2-Acetylamino-2-desoxy-D-aldohexosyl bedeutet, X Sauerstoff oder Schwefel bedeutet, Y Alkylen mit bis zu 5 C-Atomen, worin eine nicht endständige Methylengruppe durch Carbonylimino ersetzt sein kann, bedeutet, $R^2$ Wasserstoff oder Carboxy bedeutet, $R^3$ Wasserstoff und $R^4$ 1,2-Dihydroxy-ethyl, worin beide Hydroxygruppen mit einer unverzweigten $C_{10}$-$C_{20}$-Alkansäure mit gerader Anzahl von C-Atomen oder mit Oelsäure, verestert sind, bedeuten oder $R^3$ und $R^4$ je mit einer unverzweigten $C_{10}$-$C_{20}$-Alkansäure mit gerader Anzahl von C-Atomen oder mit Oelsäure verestertes Hydroxymethyl bedeuten, und ihre pharmazeutisch verwendbaren Salze.

8. Verbindungen der Formel I nach einem der Ansprüche 1-7, worin $R^1$ D-Glucosyl, 2,3,4,6-Tetra-O-acetyl-D-glucosyl, D-Mannosyl, 2,3,4,6-Tetra-O-acetyl-D-mannosyl, D-Galactosyl, D-Cellobiosyl, D-Lactosyl, D-Maltosyl, L-Fucosyl oder 2-Acetylamino-2-desoxy-D-glucosyl bedeutet, und ihre pharmazeutisch

verwendbaren Salze.

9. Ein pharmazeutisch verwendbares Salz einer Verbindung der Formel I nach Anspruch 1, worin $R^1$ 2,3,4,6-Tetra-O-acetyl-β-D-glucopyranosyl, β-D-Glucopyranosyl, 2,3,4,6-Tetra-O-acetyl-α-D-mannopyranosyl, β-D-Galactopyranosyl, β-D-Cellobiopyranosyl, β-D-Lactopyranosyl, β-D-Maltopyranosyl, β-L-Fucopyranosyl, 2-Acetylamino-2-desoxy-β-D-glucopyranosyl oder α-D-Mannopyranosyl bedeutet,
X Sauerstoff oder Schwefel bedeutet,
Y Trimethylen, Methylen oder $-CH_2-CH_2-CH_2-CONH-CH_2-$ bedeutet,
$R^2$ Wasserstoff oder Carboxy bedeutet,
$R^3$ Wasserstoff und $R^4$ 1,2-Dilauroyloxy-ethyl, 1,2-Dimyristoyloxy-ethyl, 1,2-Dipalmitoyloxy-ethyl, 1,2-Distearoyloxy-ethyl, 1,2-n-Eicosanoyloxy-ethyl, 1,2-Dioleoyloxy-ethyl, n-Decanoyloxy-methyl oder 2-Palmitoyloxy-ethyl bedeuten oder $R^3$ und $R^4$ je Lauroyloxymethyl bedeuten.

10. Ein pharmazeutisch verwendbares Salz einer Verbindung der Formel I nach einem der Ansprüche 1-9, worin $R^2$ Wasserstoff bedeutet.

11. Ein pharmazeutisch verwendbares Salz einer Verbindung der Formel I nach einem der Ansprüche 1-10, worin Y Trimethylen oder den Rest $-CH_2-CH_2-CH_2-CO-NH-CH_2-$ bedeutet.

12. Ein pharmazeutisch verwendbares Salz einer Verbindung der Formel I nach einem der Ansprüche 1-1, worin $R^3$ Wasserstoff und $R^4$ 1,2-Dipalmitoyloxy-ethyl oder $R^3$ und $R^4$ je Lauroyloxymethyl bedeuten.

13. Ein pharmazeutisch verwendbares Salz nach Anspruch 1 einer Verbindung der Formel I, ausgewählt aus 4-(2,3,4,6-Tetra-O-acetyl-β-D-glucopyranosyloxy)-buttersäure-2-(1,2-dipalmitoyl-sn-glycero-3-hydroxyphosphoryloxy)-ethylamid, 4-(β-D-Glucopyranosyloxy)-buttersäure-2-(1,2-dipalmitoyl-sn-glycero-3-hydroxyphosphoryloxy)-ethylamid, 4-(2,3,4,6-Tetra-O-acetyl-α-D-mannopyranosyloxy)-buttersäure--2-(1,2-dipalmitoyl-sn-glycero-3-hydroxyphosphoryloxy)-ethylamid, 4-(β-D-Galactopyranosyloxy)-buttersäure-2-(1,2-dipalmitoyl-sn-glycero-3-hydroxyphosphoryloxy)-ethylamid, 4-(β-D-Cellobiopyranosyloxy)-buttersäure-2-(1,2-dipalmitoyl-sn-glycero-3-hydroxyphosphoryloxy)-ethylamid, 4-(β-D-Lactopyranosyloxy)-buttersäure-2-(1,2-dipalmitoyl-sn-glycero-3-hydroxyphosphoryloxy)-ethylamid, 4-(β-D-Maltopyranosyloxy)-buttersäure-2-(1,2-dipalmitoyl-sn-glycero-3-hydroxyphosphoryloxy)-ethylamid, 4-(β-L-Fucopyranosyloxy)-buttersäure-2-(1,2-dipalmitoyl-sn-glycero-3-hydroxyphosphoryloxy)-ethylamid, N-[4-(β-D-Galactopyranosyloxy)-butyryl]-glycin-2-(1,2-dipalmitoyl-sn-glycero-3-hydroxyphosphoryloxy)-ethylamid, 4-(β-D- Cellobiopyranosyloxy)-buttersäure-2-(1,3-di-n-dodecanoyl-glycero-2-hydroxyphosphoryloxy)-ethylamid, 4-(α-D-Mannopyranosylthio)-buttersäure-2-(1,2-dipalmitoyl-sn-glycero-3-hydroxyphosphoryloxy)-ethylamid und 2-(2-Acetylamino-2-desoxy-β-D-glucopyranosyloxy)-essigsäure-2-(1,2-dipalmitoyl-sn-glycero-3-hydroxyphosphoryloxy)-ethylamid.

14. Eine Verbindung nach einem der Ansprüche 1-13 zur Anwendung in einem Verfahren zur therapeutischen Behandlung des menschlichen oder tierischen Körpers.

15. Ein pharmazeutisches Präparat, das eine Verbindung nach einem der Ansprüche 1-13 zusammen mit einem pharmazeutischen Trägermaterial enthält.

16. Verwendung einer Verbindung nach einem der Ansprüche 1-13 zur Herstellung von pharmazeutischen Präparaten, die für die Anwendung zur Prophylaxe und Therapie von Virusinfektionen bestimmt sind.

17. Verwendung nach Anspruch 16 einer Verbindung nach einem der Ansprüche 1-13 zur Herstellung von pharmazeutischen Präparaten, die für die Anwendung zur Prophylaxe und Therapie von Virusinfektionen, die durch Influenza-, Parainfluenza-, Herpes-, Encephalomyocarditis-oder Vaccinia-Viren hervorgerufen werden, bestimmt sind.

18. Verfahren zur Herstellung eines Saccharidderivats der Formel I

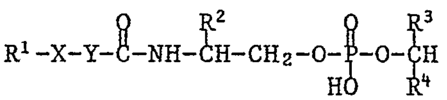

(I)

worin $R_1$ a) Aldohexosyl, b) D-Aldohexosyl, welches in 4- oder 6-Stellung glykosidisch mit D-Aldohexosyl verknüpft ist, c) Aldopentosyl, d) 6-Desoxy-aldohexosyl oder e) 2-Acetylamino-2-desoxy-D-aldohexosyl, wobei in den oben unter a) bis e) genannten Resten vorhandene freie Hydroxygruppen peracetyliert sein können, bedeutet, X Sauerstoff oder Schwefel bedeutet, Y Alkylen mit bis zu 10 C-Atomen, worin 1-3 nicht-endständige Methylengruppen durch Sauerstoff, Carbonylimino oder Carbonyloxy ersetzt sein können, bedeutet, $R^2$ Wasserstoff, Carboxy, Niederalkoxycarbonyl, Benzyloxycarbonyl oder Carbamoyl bedeutet, $R^3$ Wasserstoff und $R^4$ eine 1,2-Dihydroxy-ethyl-, 2-Hydroxy-ethyl- oder Hydroxymethyl-Gruppe, in der mindestens eine Hydroxygruppe mit einer unsubstituierten aliphatischen $C_{10-24}$-Carbonsäure verestert ist, und in der die andere Hydroxygruppe, falls vorhanden, frei oder mit einer aliphatischen $C_{2-24}$-Carbon säure verestert ist, oder $R^3$ und $R^4$ je eine mit einer unsubstituierten aliphatischen $C_{10-24}$-Carbonsäure veresterte Hydroxymethylgruppe bedeuten, oder eines Salzes davon, dadurch gekennzeichnet, dass man

a) eine Verbindung der Formel II,

$R^1 - X^1$ (II)

worin $R^1$ die obengenannte Bedeutung hat und $X^1$ für die Gruppe X-H, worin X die obengenannte

Bedeutung hat, oder für eine nucleophile Abgangsgruppe steht, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, oder ein reaktionsfähiges Derivat davon mit einer Verbindung der Formel III,

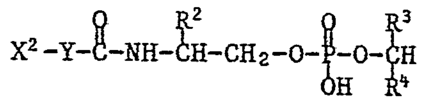
(III)

worin $X^2$ für die Gruppe H-X, worin X die obengenannte Bedeutung hat, oder für eine nucleophile Abgangsgruppe steht, und die übrigen Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, umsetzt und vorhandene Schutzgruppen abspaltet, oder

b) eine Carbonsäure der Formel IV,

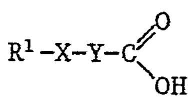
(IV)

worin die Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, oder ein reaktionsfähiges Carbonsäurederivat davon mit einer Aminoverbindung der Formel V,

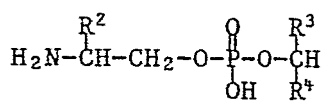
(V)

worin die Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, oder mit einem reaktionsfähigen Derivat davon umsetzt und vorhandene Schutzgruppen abspaltet, oder

c) zur Herstellung einer Verbindung der Formel I, worin Y für Alkylen steht, worin eine nicht-endständige Methylengruppe durch Carbonylimino ersetzt ist, eine Verbindung der Formel VI,

$R^1$-X-$Y^1$-$Z^1$ (VI)

worin $Y^1$ Alkylen und $Z^1$ Carboxy oder Amino bedeuten und die übrigen Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, oder ein reaktionsfähiges Derivat davon mit einer Verbindung der Formel VII,

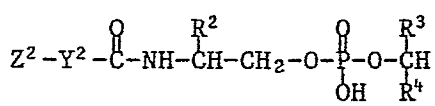
(VII)

worin $Z^2$ Amino bedeutet, wenn im Reaktionspartner der Formel VI $Z^1$ für Carboxy steht, oder worin $Z^2$ Carboxy bedeutet, wenn im Reaktionspartner der Formel VI $Z^1$ für Amino steht, und worin $Y^2$ Alkylen bedeutet, mit der Massgabe, dass der Rest -$Y^1$-CONH-$Y^2$- bzw. -$Y^1$-NHCO-$Y^2$- dem Rest Y entspricht, und die übrigen Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, oder mit einem reaktionsfähigen Derivat davon umsetzt und vorhandene Schutzgruppen abspaltet, oder

d) eine Verbindung der Formel VIII,

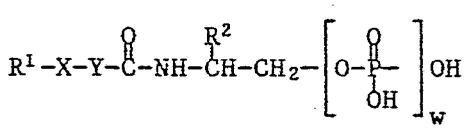
(VIII)

worin w für 0 oder 1 steht und die Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, oder ein reaktionsfähiges Derivat davon mit einer Verbindung der Formel IX,

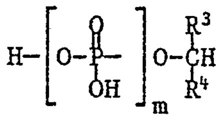 (IX)

worin m für 1 steht, wenn im Reaktionspartner der Formel VIII w für 0 steht, oder worin m für 0 steht, wenn im Reaktionspartner der Formel VIII w für 1 steht, und die Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, oder mit einem reaktionsfähigen Derivat davon umsetzt und vorhandene Schutzgruppen abspaltet, oder

e) eine Verbindung der Formel X,

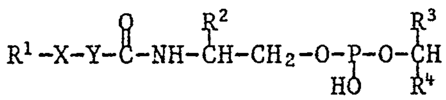 (X)

worin die Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, oder eine tautomere Verbindung mit einem geeigneten Oxidationsmittel oxidiert und vorhandene Schutzgruppen abspaltet, oder

f) eine Verbindung der Formel XI,

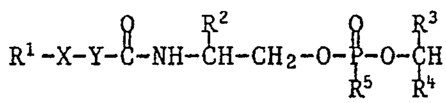 (XI)

worin $R^5$ für Halogen oder eine andere leicht hydrolysierbare Gruppe steht und die übrigen Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, hydrolysiert und vorhandene Schutzgruppen abspaltet, oder

g) eine Verbindung der Formel XII,

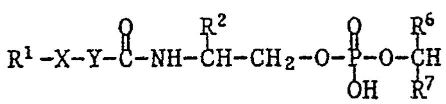 (XII)

worin $R^6$ Wasserstoff und $R^7$ 1,2-Dihydroxyethyl, 2-Hydroxy-ethyl, Hydroxymethyl oder 1,2-Dihydroxy-ethyl, worin eine der beiden Hydroxygruppen mit einer unsubstituierten aliphatischen $C_{2-24}$-Carbonsäure verestert ist, oder $R^6$ Hydroxymethyl und $R^7$ Hydroxymethyl oder Hydroxymethyl, das mit einer unsubstituierten aliphatischen $C_{10-24}$-Carbonsäure verestert ist, bedeuten, und die übrigen Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, mit einer unsubstituierten aliphatischen $C_{10-24}$-Carbonsäure oder einem reaktionsfähigen Säurederivat davon verestert und vorhandene Schutzgruppen abspaltet, oder

h) aus einer Verbindung der Formel XIII,

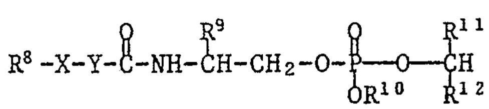 (XIII)

worin $R^8$ Aldohexosyl oder D-Aldohexosyl, welches in 4- oder 6-Stellung glykosidisch mit

D-Aldohexosyl verknüpft ist, Aldopentosyl, 6-Desoxy-aldohexosyl oder 2-Acetylamino-2-desoxy-D-aldohexosyl, wobei in den obengenannten Resten mindestens eine Hydroxygruppe durch eine leicht abspaltbare Hydroxyschutzgruppe geschützt ist, bedeutet oder $R^8$ die Bedeutung von $R^1$ hat, X und Y die obengenannten Bedeutungen haben, $R^9$ durch eine leicht abspaltbare Schutzgruppe geschütztes Carboxy bedeutet oder die Bedeutung von $R^2$ hat, $R^{10}$ Wasserstoff oder eine leicht abspaltbare Phosphorsäureschutzgruppe bedeutet, $R^{11}$ Wasserstoff bedeutet und $R^{12}$ eine 1,2-Dihydroxy-ethylgruppe, worin eine Hydroxygruppe durch eine leicht abspaltbare Schutzgruppe geschützt ist und die andere Hydroxygruppe mit einer unsubstituierten aliphatischen $C_{10-24}$-Carbonsäure verestert ist, bedeutet oder $R^{12}$ die Bedeutung von $R^4$ hat, mit der Massgabe, dass in einer Verbindung der Formel XIII mindestens eine leicht abspaltbare Schutzgruppe vorhanden ist, die in dem gewünschten Endprodukt der Formel I nicht enthalten ist, diese Schutzgruppe(n) abspaltet, und nach Ausführung eines der obengenannten Verfahren a)-h) zur Herstellung eines Salzes erforderlichenfalls eine Verbindung der Formel I in eines ihrer Salze überführt.

Patentanspüche für die folgenden Vertragsstaaten At, ES und GR

1. Verfahren zur Herstellung eines Saccharidderivats der Formel I

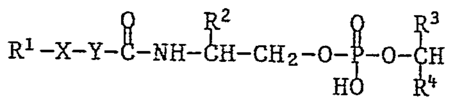

(I)

worin $R_1$ a) Aldohexosyl, b) D-Aldohexosyl, welches in 4- oder 6-Stellung glykosidisch mit D-Aldohexosyl verknüpft ist, c) Aldopentosyl, d) 6-Desoxy-aldohexosyl oder e) 2-Acetylamino-2-desoxy-D-aldohexosyl, wobei in den oben unter a) bis e) genannten Resten vorhandene freie Hydroxygruppen peracetyliert sein können, bedeutet, X Sauerstoff oder Schwefel bedeutet, Y Alkylen mit bis zu 10 C-Atomen, worin 1-3 nicht-endständige Methylengruppen durch Sauerstoff, Carbonylimino oder Carbonyloxy ersetzt sein können, bedeutet, $R^2$ Wasserstoff, Carboxy, Niederalkoxycarbonyl, Benzyloxycarbonyl oder Carbamoyl bedeutet, $R^3$ Wasserstoff und $R^4$ eine 1,2-Dihydroxy-ethyl-, 2-Hydroxyethyl- oder Hydroxymethyl-Gruppe, in der mindestens eine Hydroxygruppe mit einer unsubstituierten aliphatischen $C_{10-24}$-Carbonsäure verestert ist, und in der die andere Hydroxygruppe, falls vorhanden, frei oder mit einer aliphatischen $C_{2-24}$-Carbonsäure verestert ist, oder $R^3$ und $R^4$ je eine mit einer unsubstituierten aliphatischen $C_{10-24}$-Carbonsäure veresterte Hydroxymethylgruppe bedeuten, oder eines Salzes davon, dadurch gekennzeichnet, dass man

a) eine Verbindung der Formel II,

$R^1$ - $X^1$ (II)

worin $R^1$ die obengenannte Bedeutung hat und $X^1$ für die Gruppe X-H, worin X die obengenannte Bedeutung hat, oder für eine nucleophile Abgangsgruppe steht, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, oder ein reaktionsfähigen Derivat davon mit einer Verbindung der Formel III,

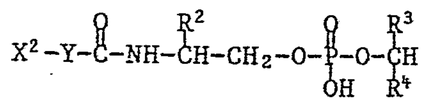

(III)

worin $X^2$ für die Gruppe H-X, worin X die obengenannte Bedeutung hat, oder für eine nucleophile Abgangsgruppe steht, und die übrigen Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, umsetzt und vorhandene Schutzgruppen abspaltet, oder

b) eine Carbonsäure der Formel IV,

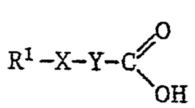

(IV)

worin die Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, oder ein reaktionsfähiges Carbonsäurederivat davon mit einer Aminoverbindung der Formel V,

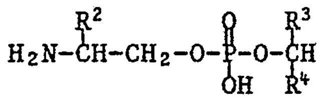 (V)

worin die Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, oder mit einem reaktionsfähigen Derivat davon umsetzt und vorhandene Schutzgruppen abspaltet, oder

c) zur Herstellung einer Verbindung der Formel I, worin Y für Alkylen steht, worin eine nicht-endständige Methylengruppe durch Carbonylimino ersetzt ist, eine Verbindung der Formel VI,
$R^1$-X-$Y^1$-$Z^1$ (VI)
worin $Y^1$ Alkylen und $Z^1$ Carboxy oder Amino bedeuten und die übrigen Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, oder ein reaktionsfähiges Derivat davon mit einer Verbindung der Formel VII,

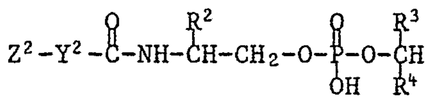 (VII)

worin $Z^2$ Amino bedeutet, wenn im Reaktionspartner der Formel VI $Z^1$ für Carboxy steht, oder worin $Z^2$ Carboxy bedeutet, wenn im Reaktionspartner der Formel VI $Z^1$ für Amino steht, und worin $Y^2$ Alkylen bedeutet, mit der Massgabe, dass der Rest -$Y^1$-CONH-$Y^2$- bzw. -$Y^1$-NHCO-$Y^2$- dem Rest Y entspricht, und die übrigen Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, oder mit einem reaktionsfähigen Derivat davon umsetzt und vorhandene Schutzgruppen abspaltet, oder

d) eine Verbindung der Formel VIII,

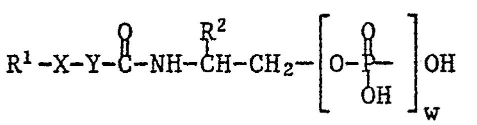 (VIII)

worin w für 0 oder 1 steht und die Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, oder ein reaktionsfähiges Derivat davon mit einer Verbindung der Formel IX,

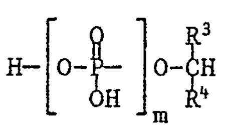 (IX)

worin m für 1 steht, wenn im Reaktionspartner der Formel VIII w für 0 steht, oder worin m für 0 steht, wenn im Reaktionspartner der Formel VIII w für 1 steht, und die Substituenten die obengenannten Bedeutungen haben, wobei in dieser Verbindung vorhandene freie funktionelle Grupen mit Ausnahme der an der Reaktion teilnehmenden Gruppe erforderlichenfalls durch leicht abspaltbare Schutzgruppen geschützt sind, oder mit einem reaktionsfähigen Derivat davon umsetzt und vorhandene Schutzgruppen abspaltet, oder

e) eine Verbindung der Formel X,

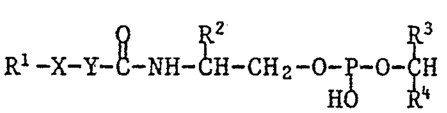 (X)

worin die Substituenten die obengenannten Bedeutungen haben, wobei in dieser Verbindung vorhandene freie funktionelle Gruppen mit Ausnahme der an der Reaktion teilnehmenden Gruppe

erforderlichenfalls durch leicht abspaltbare Schutzgruppen geschützt sind, oder eine tautomere Verbindung mit einem geeigneten Oxidationsmittel oxidiert und vorhandene Schutzgruppen abspaltet, oder

f) eine Verbindung der Formel XI,

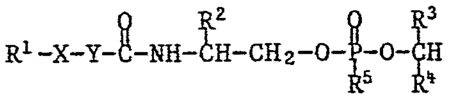

(XI)

worin $R^5$ für Halogen oder eine andere leicht hydrolysierbare Gruppe steht und die übrigen Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, hydrolysiert und vorhandene Schutzgruppen abspaltet, oder

g) eine Verbindung der Formel XII,

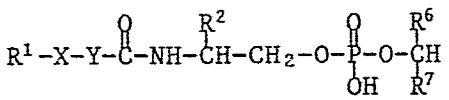

(XII)

worin $R^6$ Wasserstoff und $R^7$ 1,2-Dihydroxyethyl, 2-Hydroxy-ethyl, Hydroxymethyl oder 1,2-Dihydroxy-ethyl, worin eine der beiden Hydroxygruppen mit einer unsubstituierten aliphatischen $C_{2-24}$-Carbonsäure verestert ist, oder $R^6$ Hydroxymethyl und $R^7$ Hydroxymethyl oder Hydroxymethyl, das mit einer unsubstituierten aliphatischen $C_{10-24}$-Carbonsäure verestert ist, bedeuten, und die übrigen Substituenten die obengenannten Bedeutungen haben, wobei in dieser Verbindung vorhandene freie funktionelle Gruppen mit Ausnahme der an der Reaktion teilnehmenden Gruppe erforderlichenfalls durch leicht abspaltbare Schutzgruppen geschützt sind, mit einer unsubstituierten aliphatischen $C_{10-24}$-Carbonsäure oder einem reaktionsfähigen Säurederivat davon verestert und vorhandene Schutzgruppen abspaltet, oder

h) aus einer Verbindung der Formel XIII,

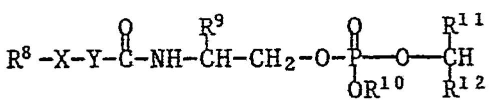

(XIII)

worin $R^8$ Aldohexosyl oder D-Aldohexosyl, welches in 4- oder 6-Stellung glykosidisch mit D-Aldohexosyl verknüpft ist, Aldopentosyl, 6-Desoxy-aldohexosyl oder 2-Acetylamino-2-desoxy-D-aldohexosyl, wobei in den obengenannten Resten mindestens eine Hydroxygruppe durch eine leicht abspaltbare Hydroxyschutzgruppe geschützt ist, bedeutet oder $R^8$ die Bedeutung von $R^1$ hat, X und Y die obengenannten Bedeutungen haben, $R^9$ durch eine leicht abspaltbare Schutzgruppe geschütztes Carboxy bedeutet oder die Bedeutung von $R^2$ hat, $R^{10}$ Wasserstoff oder eine leicht abspaltbare Phosphorsäureschutzgruppe bedeutet, $R^{11}$ Wasserstoff bedeutet und $R^{12}$ eine 1,2-Dihydroxy-ethylgruppe, worin eine Hydroxygruppe durch eine leicht abspaltbare Schutzgruppe geschützt ist und die andere Hydroxygruppe mit einer unsubstituierten aliphatischen $C_{10-24}$-Carbonsäure verestert ist, bedeutet oder $R^{12}$ die Bedeutung von $R^4$ hat, mit der Massgabe, dass in einer Verbindung der Formel XIII mindestens eine leicht abspaltbare Schutzgruppe vorhanden ist, die in dem gewünschten Endprodukt der Formel I nicht enthalten ist, diese Schutzgruppe(n) abspaltet, und nach Ausführung eines der obengenannten Verfahren a)-h) zur Herstellung eines Salzes erforderlichenfalls eine Verbindung der Formel I in eines ihrer Salze überführt.

2. Verfahren nach Anspruch 1, dadurch gekennzeichnet, dass man die Ausgangsstoffe so wählt, dass man eine Verbindung der Formel I, worin $R^3$ Wasserstoff und $R^4$ 1,2-Dihydroxy-ethyl, worin entweder nur die 2-Hydroxy-Gruppe oder beide Hydroxygruppen unabhängig voneinander mit einer $C_{10-24}$-Alkansäure oder einer $C_{18}$-Alkensäure verestert sind, bedeuten oder $R^3$ und $R^4$ je mit einer $C_{10-24}$-Alkansäure oder einer $C_{18}$-Alkensäure verestertes Hydroxymethyl bedeuten und die übrigen Substituenten die in Anspruch 1 genannten Bedeutungen haben, oder ein Salz davon herstellt.

3. Verfahren nach Anspruch 1, dadurch gekennzeichnet, dass man die Ausgangsstoffe so wählt, dass man eine Verbindung der Formel I, worin $R^3$ Wasserstoff und $R^4$ 1,2-Dihydroxyethyl, worin beide Hydroxygruppen unabhängig voneinander mit einer $C_{10-24}$-Alkansäure oder einer $C_{18}$-Alkensäure verestert sind, bedeuten oder $R^3$ und $R^4$ je mit einer $C_{10-24}$-Alkansäure oder einer $C_{18}$-Alkensäure verestertes Hydroxymethyl bedeuten und die übrigen Substituenten die in Anspruch 1 genannten Bedeutungen haben, oder ein Salz davon herstellt.

4. Verfahren nach Anspruch 1, dadurch gekennzeichnet, dass man die Ausgangsstoffe so wählt, dass

man eine Verbindung der Formel I, worin $R^3$ Wasserstoff und $R^4$ 1,2-Dihydroxyethyl, worin beide Hydroxygruppen unabhängig voneinander mit einer unverzweigten $C_{10-24}$-Alkansäure mit gerader Anzahl von C-Atomen oder einer unverzweigten $C_{18}$-Alkensäure mit 1-3 isolierten Doppelbindungen verestert sind, bedeuten oder $R^3$ und $R^4$ je mit einer unverzweigten $C_{10-24}$-Alkansäure mit gerader Anzahl von C-Atomen oder einer unverzweigten $C_{18}$-Alkensäure mit 1-3 isolierten Doppelbindungen verestertes Hydroxymethyl bedeuten und die übrigen Substituenten die in Anspruch 1 genannten Bedeutungen haben, oder ein Salz davon herstellt.

5. Verfahren nach einem der Ansprüche 1-4, dadurch gekennzeichnet, dass man die Ausgangsstoffe so wählt, dass man eine Verbindung der Formel I, worin Y unverzweigtes $C_1$-$C_5$-Alkylen oder den Rest $-CH_2-CH_2-CH_2-CO-NH-CH_2-$bedeutet, oder ein Salz davon herstellt.

6. Verfahren nach Anspruch 1, dadurch gekennzeichnet, dass man die Ausgangsstoffe so wählt, dass man eine Verbindung der Formel I, worin $R^1$ a) D-Aldohexosyl, welches in 4-Stellung glykosidisch mit D-Aldohexosyl verknüpft sein kann, b) 6-Desoxy-aldohexosyl oder c) 2-Acetylamino-2-desoxy-D-aldohexosyl, wobei in den oben unter a) bis c) genannten Resten vorhandene freie Hydroxygruppen peracetyliert sein können, bedeutet, X Sauerstoff oder Schwefel bedeutet, Y $C_{3-5}$-Alkylen, worin eine nicht endständige Methylengruppe durch Carbonylimino ersetzt sein kann, bedeutet, $R^2$ Wasserstoff oder Carboxy bedeutet, $R^3$ Wasserstoff und $R^4$ 1,2-Dihydroxy-ethyl, worin entweder nur die 2-Hydroxy-Gruppe oder beide Hydroxygruppen unabhängig voneinander mit einer unverzweigten $C_{10-24}$-Alkansäure oder einer unverzweigten $C_{18}$-Alkensäure verestert sind, bedeuten oder $R^3$ und $R^4$ je mit einer unverzweigten $C_{10-24}$-Alkansäure oder einer unverzweigten $C_{18}$-Alkensäure mit 1-3 isolierten Doppelbindungen verestertes Hydroxymethyl bedeuten, oder ein Salz davon herstellt.

7. Verfahren nach Anspruch 1, dadurch gekennzeichnet, dass man die Ausgangsstoffe so wählt, dass man eine Verbindung der Formel I, worin $R^1$ a) D-Aldohexosyl, worin freie Hydroxygruppen peracetyliert sein können, b) D-Aldohexosyl, welches in 4-Stellung glykosidisch mit D-Aldohexosyl verknüpft ist, c) 6-Desoxy-aldohexosyl oder d) 2-Acetylamino-2-desoxy-D-aldohexosyl bedeutet, X Sauerstoff oder Schwefel bedeutet, Y Alkylen mit bis zu 5 C-Atomen, worin eine nicht endständige Methylengruppe durch Carbonylimino ersetzt sein kann, bedeutet, $R^2$ Wasserstoff oder Carboxy bedeutet, $R^3$ Wasserstoff und $R^4$ 1,2-Dihydroxy-ethyl, worin beide Hydroxygruppen mit einer unverzweigten $C_{10-20}$-Alkansäure mit gerader Anzahl von C-Atomen oder mit Oelsäure, verestert sind, bedeuten oder $R^3$ und $R^4$ je mit einer unverzweigten $C_{10}$-$C_{20}$-Alkansäure mit gerader Anzahl von C-Atomen oder mit Oelsäure verestertes Hydroxymethyl bedeuten, oder ein pharmazeutisch verwendbares Salz davon herstellt.

8. Verfahren nach einem der Ansprüche 1-7, dadurch gekennzeichnet, dass man die Ausgangsstoffe so wählt, dass man eine Verbindung der Formel I, worin $R^1$ D-Glucosyl, 2,3,4,6-Tetra-O-acetyl-D-glucosyl, D-Mannosyl, 2,3,4,6-Tetra-O-acetyl-D-mannosyl, D-Galactosyl, D-Cellobiosyl, D-Lactosyl, D-Maltosyl, L-Fucosyl oder 2-Acetylamino-2-desoxy-D-glucosyl bedeutet, oder ein pharmazeutisch verwendbares Salz davon herstellt.

9. Verfahren nach Anspruch 1, dadurch gekennzeichnet, dass man ein pharmazeutisch verwendbares Salz einer Verbindung der Formel I, worin $R^1$ 2,3,4,6-Tetra-O-acetyl-β-D-glucopyranosyl, β-D-Glucopyranosyl, 2,3,4,6-Tetra-O-acetyl-α-D-mannopyranosyl, β-D-Galactopyranosyl, β-D-Cellobiopyranosyl, β-D-Lactopyranosyl, β-D-Maltopyranosyl, β-L-Fucopyranosyl, 2-Acetylamino-2-desoxy-β-D-glucopyranosyl oder α-D-Mannopyranosyl bedeutet,
X Sauerstoff oder Schwefel bedeutet,
Y Trimethylen, Methylen oder $-CH_2-CH_2-CH_2-CONH-CH_2-$ bedeutet,
$R^2$ Wasserstoff oder Carboxy bedeutet,
$R^3$ Wasserstoff und $R^4$ 1,2-Dilauroyloxy-ethyl, 1,2-Dimyristoyloxy-ethyl, 1,2-Dipalmitoyloxy-ethyl, 1,2-Distearoyloxy-ethyl, 1,2-n-Eicosanoyloxy-ethyl, 1,2-Dioleoyloxy-ethyl, n-Decanoyloxy-methyl oder 2-Palmitoyloxy-ethyl bedeuten oder $R^3$ und $R^4$ je Lauroyloxymethyl bedeuten, herstellt.

10. Verfahren nach einem der Ansprüche 1-9, dadurch gekennzeichnet, dass man ein pharmazeutisch verwendbares Salz einer Verbindung der Formel I, worin $R^2$ Wasserstoff bedeutet, herstellt.

11. Verfahren nach einem der Ansprüche 1-10, dadurch gekennzeichnet, dass man ein pharmazeutisch verwendbares Salz einer Verbindung der Formel I, worin Y Trimethylen oder den Rest $-CH_2-CH_2-CH_2-CO-NH-CH_2-$bedeutet, herstellt.

12. Verfahren nach einem der Ansprüche 1-11, dadurch gekennzeichnet, dass man ein pharmazeutisch verwendbares Salz einer Verbindung der Formel I, worin $R^3$ Wasserstoff und $R^4$ 1,2-Dipalmitoyloxy-ethyl oder $R^3$ und $R^4$ je Lauroyloxymethyl bedeuten, herstellt.

13. Verfahren nach Anspruch 1, dadurch gekennzeichnet, dass man ein pharmazeutisch verwendbares Salz einer Verbindung der Formel I, ausgewählt aus 4-(2,3,4,6-Tetra-O-acetyl-β-D-glucopyranosyloxy)-buttersäure-2-(1,2-dipalmitoyl-sn-glycero-3-hydroxyphosphoryloxy)-ethylamid, 4-(β-D-Glucopyranosyloxy)-buttersäure-2-(1,2-dipalmitoyl-sn-glycero-3-hydroxyphosphoryloxy)-ethylamid, 4-(2,3,4,6-Tetra-O-acetyl-α-D-mannopyranosyloxy)-buttersäure-2-(1,2-dipalmitoyl-sn-glycero-3-hydroxyphosphoryloxy)-ethylamid, 4-(β-D-Galactopyranosyloxy)-buttersäure-2-(1,2-dipalmitoyl-sn-glycero-3-hydroxyphosphoryloxy)-ethylamid, 4-(β-D-Cellobiopyranosyloxy)-buttersäure-2-(1,2-dipalmitoyl-sn-glycero-3-hydroxyphosphoryloxy)-ethylamid, 4-(β-D-Lactopyranosyloxy)-buttersäure-2-(1,2-dipalmitoyl-sn-glycero-3-hydroxyphosphoryloxy)-ethylamid, 4-(β-D-Maltopyranosyloxy)-buttersäure-2-(1,2-dipalmitoyl-sn-glycero-3-hydroxyphosphoryloxy)-ethylamid, 4-(β-L-Fucopyranosyloxy)-buttersäure-2-(1,2-dipalmitoyl-

sn-glycero-3-hydroxyphosphoryloxy)-ethylamid, N-[4-(β-D-Galactopyranosyloxy)-butyryl]-glycin-2-(1,2-dipalmitoyl-sn-glycero-3-hydroxyphosphoryloxy)-ethylamid, 4-(β-D-Cellobiopyranosyloxy)-buttersäure-2-(1,3-di-n-dodecanoyl-glycero-2-hydroxyphosphoryloxy)-ethylamid, 4-(α-D-Mannopyranosylthio)-buttersäure-2-(1,2-dipalmitoyl-sn-glycero-3-hydroxyphosphoryloxy)-ethylamid und 2-(2-Acetylamino-2-desoxy-β-D-glucopyranosyloxy)-essigsäure-2-(1,2-dipalmitoyl-sn-glycero-3-hydroxyphosphoryloxy)-ethylamid, herstellt.